# FASCICULE DE BREVET EUROPEEN

(11) **EP 2 826 460 B1**
(45) Date de publication et mention de la délivrance du brevet: **23.08.2017**
(21) Numéro de dépôt: 14177909.0
(22) Date de dépôt: 02.07.2010
(51) Int. Cl.: A61K 8/11, A61K 8/20, A61K 8/29, A61K 8/37, A61K 8/49, A61Q 1/02, A61K 8/891, A61K 8/58, A61Q 19/00, A61K 8/04, A61K 8/06, A61Q 1/12

(54) **Emulsion contenant une dispersion d'oxychlorure de bismuth**
Emulsion, die eine Bismutchloridoxid-Dispersion enthält
Emulsion containing a dispersion of bismuth oxychloride

(30) Priorité: 20.07.2009 FR 0955009; 20.07.2009 FR 0955006; 20.07.2009 FR 0955008; 22.09.2009 US 244457 P; 22.09.2009 US 244458 P; 22.09.2009 US 244461 P; 02.06.2010 FR 1054303
(43) Date de publication de la demande: 21.01.2015
(62) Demande divisionnaire de: 10742183.6
(73) Titulaire: L'OREAL, 75008 Paris (FR)
(72) Inventeur: Ozee, Emmanuelle, 94320 THIAIS (FR); Guerchet, Laurence, 33110 LE BOUSCAT (FR); Blin, Xavier, 75015 PARIS (FR); Delattre, Nathalie, 78120 SONCHAMP (FR)
(74) Mandataire: Goudet, Sylvain

(56) Documents cités:
- WO-A1-2005/000252
- WO-A2-01/51017
- WO-A2-2008/079760
- FR-A1- 2 903 006
- US-A1- 2007 048 238
- US-B1- 6 743 285
- "Formulation Example IPL-07-14-1 Pearly Lipstick Fresh Watermelon", INTERNET CITATION, 1 juin 2009 (2009-06-01), page 1, XP002567304, Extrait de l'Internet: URL:http://www.merck-chemicals.de/cosmetic -ingredients/timiron-liquid-sil ver/MDA_CHEM-117021/p_0qab.s1Lh2UAAAEWPJwf VhTm?attachments=TI [extrait le 2010-02-09]
- "Formulation Example IPP-09-01-3 Creamy Eyeshadow Turquoise", INTERNET CITATION, 1 juin 2009 (2009-06-01), page 1, XP002567305, Extrait de l'Internet: URL:http://www.merck-chemicals.de/cosmetic -ingredients/timiron-liquid-sil ver/MDA_CHEM-117021/p_0qab.s1Lh2UAAAEWPJwf VhTm?attachments=TI [extrait le 2010-02-09]

## Description

La présente invention concerne des compositions de soin et/ou de maquillage de la peau, destinées notamment à conférer de la luminosité ou un effet de lumière.

Par 'luminosité' ou 'effet de lumière', on entend selon l'invention, la caractéristique de reflexion de la lumière, réflexion diffuse et continue sur la peau. En effet, la peau réfléchit naturellement une partie de la lumière incidente. L'« effet lumière » selon l'invention, permet d'augmenter cette réflexion, ce qui offre au maquillage un rendu plus lumineux, plus d'éclat. Les compositions selon l'invention peuvent en outre conférer un effet bonne mine.

Par 'effet bonne mine', on entend une coloration naturelle de la peau, avec une amélioration de l'aspect terne du teint (effet désaturant ou chromatique et anti-teint terne).

Par composition de soin et/ou de maquillage des matières kératiniques selon l'invention, on entend des compositions distinctes des compositions de nettoyage à rincer.

En particulier, il s'agira de compositions de soin et/ou de maquillage de la peau, du visage et/ou du corps, notamment du visage.

Selon un mode particulier, la composition selon l'invention comprendra au moins une matière colorante.

Les consommateurs sont à la recherche de nouveaux produits cosmétiques destinés à améliorer l'aspect des matières kératiniques et notamment la peau, en particulier l'aspect de surface (irrégularités visibles et/ou tactiles) et/ou le teint de la peau, signe extérieur de bonne mine, santé et jeunesse.

Par ailleurs, ils sont à la recherche de nouveaux produits cosmétiques présentant une texture fluide, fraîche, et fine associée à un résultat de soin et/ou de maquillage fin, non poudreux, non marquant mais néanmoins couvrant et lumineux pour performer dans l'atténuation des défauts, en particulier l'aspect de surface (irrégularités visibles et/ou tactiles), et apporter de la luminosité au teint, signe extérieur de bonne mine, santé et jeunesse.

Ces textures fluides, notamment recherchées pour les peaux grasses et dans les pays chauds, sont néanmoins plus difficiles à stabiliser, notamment lorsqu'elles contiennent un fort taux d'eau et d'alcools (pour l'effet frais), associé à la présence de charges et de pigments (pour l'effet couvrance et l'effet coloriel). Ceci est d'autant plus vrai pour les émulsions E/H, classiques dans le domaine des fond de teint.

Par ailleurs, ces formules fraîches sont avantageusement peu chargées au sens de contenant peu de charges ; cependant il est important que ces formules soient couvrantes pour masquer les défauts (dyschromies, taches, irrégularités de la peau). C'est particulièrement vrai en Asie, au Japon où, tout en devant être fraiches, légères et fines, les textures doivent apporter de la couvrance. Il est donc important de disposer dans ces formules de matériaux qui savent, sans destabiliser et sans épaissir ces textures, apporter de la couvrance mais aussi de la luminosité.

D'autre part, les consommateurs sont également à la recherche de nouveaux produits cosmétiques destinés à améliorer l'aspect des matières kératiniques et notamment la peau, en particulier l'aspect de surface (irrégularités visibles et/ou tactiles) et/ou le teint de la peau, signe extérieur de bonne mine, santé et jeunesse.

Au cours du processus de vieillissement par exemple, la structure de la peau et ses fonctions cutanées se modifient : les principaux signes cliniques du vieillissement cutané sont notamment l'apparition de ridules et de rides profondes, en augmentation avec l'âge. On constate également une désorganisation du "grain" de la peau, c'est-à-dire que le micro-relief est moins régulier et présente un caractère anisotrope.

Il est connu de traiter ces signes du vieillissement en utilisant des compositions cosmétiques ou dermatologiques contenant des actifs capables de lutter contre le vieillissement. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés.

D'autres méthodes connues pour camoufler les défauts de la peau utilisent des compositions contenant des charges dites à effet de flou ou « soft-focus » telles que décrites dans la demande EP-A-1099437, qui atténuent par effet optique les défauts cutanés tels que les taches, les rides, les ridules.

Toutefois, les résultats obtenus avec ces compositions sont souvent insuffisants pour atténuer voire masquer efficacement les rides les plus prononcées. En outre, l'effet soft focus est souvent altéré par la présence de charges opaques ou de pigments dans les compositions. De plus, les particules solides comme les charges, les matières colorantes pulvérulentes présentes dans les compositions de maquillage de la peau présentent l'inconvénient de se concentrer dans les rides, en particulier les rides profondes, accentuant ainsi les irrégularités de la peau. L'application de ces compositions sur des peaux particulièrement ridées (notamment les peaux matures) conduit à un maquillage marquant ou révélant les rides.

Les inventeurs ont par ailleurs étudié l'impact de la lumière visible sur la peau : 5% du rayonnement réfléchisssent directement, reflétant tout le spectre de couleurs de la lumière : c'est l'éclat de surface, alors que 95% pénètrent dans la peau, interagissent avec l'épiderme et le derme, et 40% sont rediffusés en surface: c'est la lumière intérieure de la peau.

Mais lorsque la qualité de la peau s'altère (fatigue, sécheresse, vieillissement...) :
- l'éclat de surface est minimisé, car la lumière se réfléchit moins bien sur un relief irrégulier,
- la lumière intérieure perd l'intensité de son rayonnement, car elle est plus absorbée, notamment par la mélanine, dans ses composantes bleutées.

On connaît de l'art antérieur l'utilisation de charges pour conférer de la couvrance et de pigments (parmi lesquels le TiO2) qui, pour ces derniers, apportent la couleur en plus de la couvrance. Ils permettent de moduler la « couverture » visuelle de la peau par l'application d'une composition (ex : fond de teint) contenant lesdits charges/pigments et donnent ainsi aux consommateurs(trices) la possibilité :
- d'unifier la couleur du teint, voire de la rehausser lorsque la femme (l'homme) juge que sa carnation naturelle n'est pas en phase avec l'image qu'elle (il) veut donner d'elle (de lui), le climat, la saison... ; et/ou
- de corriger les défauts qui les gênent depuis toujours ou qui, l'âge venant, surviennent et qu'elle (il) souhaiterait atténuer ou cacher.

Malheureusement ces matières premières peuvent marquer le visage en soulignant le relief et le microrelief du visage. Ceci est d'autant plus dommage que l'âge est un facteur d'accentuation de ce microrelief et il n'est pas bienvenu pour ces consommateurs (trices) d'accentuer ce dernier par le même fond de teint qu'ils (elles) utilisent pour couvrir et colorer leur visage.

Par ailleurs ces mêmes matières premières, présentent aussi l'intérêt d'amener de la matité au visage mais par là-même peuvent conférer un aspect terne au teint.

Il subsiste donc le besoin de trouver de nouveaux produits de soin et/ou de maquillage, notamment pour le teint, qui donnent un effet lumineux, sans être brillant, ne marquant pas le relief tout en couvrant les défauts (dyschromies, taches) ou apportant une teinte unifiée au visage, en particulier une teinte naturelle et unifiée au visage, avantageusement un effet bonne mine.

Selon une alternative ou de façpn complémentaire, on recherche de nouveaux produits en particulier de texture fluide, fraîche, et fine, associée à un résultat de soin et/ou de maquillage fin, non poudreux, non marquant mais néanmoins couvrant et lumineux.

Selon une alternative ou de façon complémentaire, on recherche de nouveaux produits permettant d'obtenir un camouflage satisfaisant des défauts de la peau, en gardant un aspect naturel et lumineux du teint, sans être brillant.

Les inventeurs ont mis en évidence que l'utilisation d'une dispersion (autrement nommée pré-dispersion) d'oxychlorure de bismuth de l'hydroxystéarate d'éthyl (2) hexyle (nom INCI ethylhexyl hydroxystearate), jusqu'ici connue dans des compositions anhydres de vernis, gloss, fard à paupières, pour son effet brillant, permettait, en formulation dans une émulsion destinée à une application sur la peau, de conférer de la luminosité à la peau ; le teint retrouve ainsi la fraîcheur et l'éclat de la jeunesse.

Comparativement à l'utilisation de nacres ou de pigments de type dioxyde de titane utilisés classiquement pour la recherche d'un effet couvrant et lumineux, l'effet obtenu avec la dispersion d'oxychlorure de bismuth utilisée selon l'invention est avantageusement homogène, et san point scintillant.

Les inventeurs ont par ailleurs mis en évidence que l'utilisation d'une dispersion (pré-dispersion) d'oxychlorure de bismuth dans de l'hydroxystéarate d'éthyl (2) hexyle (nom INCI ethylhexyl hydroxystearate), permettait, en formulation dans une émulsion destinée à une application sur la peau et contenant un fort taux d'eau et d'alcools, de conférer de la luminosité à la peau et un effet frais ; le teint retrouve ainsi la fraîcheur et l'éclat de la jeunesse.

Les inventeurs ont également mis en évidence que l'utilisation d'une dispersion (pré-dispersion) d'oxychlorure de bismuth dans de l'hydroxystéarate d'éthyl (2) hexyle (nom INCI ethylhexyl hydroxystearate), permettait, en formulation avec un agent soft focus dans une composition destinée à une application sur la peau, de masquer les défauts cutanés en conférant de la luminosité à la peau ; le teint retrouve ainsi la fraîcheur et l'éclat de la jeunesse.

Et ils ont mis en évidence que l'utilisation de l'association d'une pré-dispersion d'oxychlorure de bismuth dans de l'hydroxystéarate d'éthyl (2) hexyle (nom INCI ethylhexyl hydroxystearate), avec une matière colorante particulière (notamment un pigment composite tel que défini ci-après), dans une composition destinée à une application sur la peau, permettait de donner de la lumière à la peau et d'améliorer son éclat de surface, vantageusement avec un effet bonne mine : le teint est plus frais, plus lumineux, plus éclatant.

L'utilisation de l'oxychlorure de bismuth (Cl 77163) sous forme de poudre ou agglomérats était connue dans les fonds de teint en tant que charge destinée à apporter une certaine sensorialité (toucher doux). Ce composé pouvait également apporter un effet satiné, ponctuel et discontinu, qui dans des produits comme les fluides, les compacts ou voire les poudres peut être perçu comme une brillance nacrée et non pas le fondu lumineux recherché.

La demande WO2004/041234 décrit par ailleurs des compositions anhydres éclaircissantes et blanhissantes comprenant extrait de *Phyllanthus emblica* (PE) et comme additif, de l'oxychlorure de bismuth sous forme de poudre ou de dispersion pour conférer à la composition un toucher sec et doux sur la peau.

La forme en dispersion (Biron® Liquid Silver) est décrite dans cette demande et commercialisée par le fournisseur Merck, comme une matière première hautement lustrante, brillante, qui ne sont pas des propriétés recherchées pour une application sur la peau du visage, et notamment pour le teint. Lorsqu'on l'observe pure, elle se présente comme un liquide très argenté et extrêmement brillant, tout indiqué et recommandé par le fournisseur pour des utilisations dans des compositions de type gloss, vernis et fard à paupières, mais *a priori* inadapté pour les attentes d'un résultat de maquillage de teint. On connaît également du brevet US 6,906,015 son utilisation dans des compositions de nettoyage à rincer.

On a déjà proposé d'utiliser une dispersion d'oxychlorure de bismuth dans dans de l'hydroxystéarate d'éthyl (2) hexyle dans des produits de maquillage comme la formulation Exemple IPL-07-14-1 « Pearly Lipstick Fresh Watermelon » juin 2009 http://www.merck-chemicals.de/cosmetic-ingredients/timiron-liquid-silver/MDA_CHEM-117021/p_Oqab.s1Lh2UAAAEWPJwfVhTm?attachements=TI ou la formulation Exemple IPP-09-01-3 « Creamy Eye Shadow Turquoise » juin 2009 http://www.merck-chemicals.de/cosmetic-ingredients/timiron-liquid-silver/MDA_CHEM-117021/p_Oqab.s1Lh2UAAAEWPJwfVhTm?attachements=TI. On connait dans la demande WO2008/079760 l'utilisation de l'oxychlorure de bismuth dans un exemple de fond de teint sous forme d'émulsion. On connait l'utilisation de l'oxychlorure de bismuth. dans la demande FR2930066 l'utilisation d'un mica-oxyde de fer brun dans des produits de maquillage et/ou de soin des peaux foncées à base d'un dérivé c-glucoside. On connaît dans la demande de brevet US2005048014 des compositions rincées à base d'une dispersion d'oxychlorure de bismuth et d'hydroxystérate d'éthyl (2) hexyle. On connaît dans la demande WO2005000252 des émulsions comprenant un agent gélifiant, une résiline siliconée polyamide et pouvant contenir de l'oxychlorure de bismuth. On connaît dans la demande EP1327435 des compositions de fards à paupières comprenant une gomme de xanthane, un silicate mixte et une matière colorante et pouvant contenir de l'oxychlorure de bismuth. On connaît dans le brevet US6743285 un mélange de prigments sous forme de dispersion comprenant un composant A contenant un pigment enrobé ou non et un composé B comprenant un pigment nacré comprenant du mica, du verre, Fe₂O₃, TiO₂, SiO₂, des plaquettes de polymère ou de graphite, des colorants et/ou des charges de forme plaquettaire, d'aiguilles ou sphériques. On connaît dans la demande US2007048238 des fonds de teints anhydres pouvant contenir de l'oxychlorure de bismuth. On connaît le document WO01/51017 décrivant un procédé de camouflage des ridules et rides comprenant l'association d'un pigment interférentiel et d'un pigment d'oxyde métallique et pouvant contenir de l'oxychlorure de bismuth.

Or nous avons mis en évidence que l'utilisation de cette dispersion (pré-dispersion) d'oxychlorure de bismuth dans de l'hydroxystéarate d'éthyl (2) hexyle pouvait amener aux produits de soin et/ou de maquillage notamment de teint, en particulier de type émulsions, la capacité d'apporter après application/maquillage :
a) de la couvrance,
b) et surtout de la luminosité.

Selon un autre mode particulier, la composition selon l'invention comprend au moins une phase aqueuse formant de 30 à 70% en poids par rapport au poids total de la composition et au moins 5% en poids par rapport au poids total de la composition, d'au moins un mono-alcools en C₂-C₈.

Selon un autre mode particulier, la composition selon l'invention comprend au moins un agent soft focus tel que défini ci-après.

Selon un mode particulier, la composition selon l'invention comprend au moins une matière colorante choisie parmi les agents de coloration goniochromatiques, les agents de coloration photochromes, les agents fluorescents, les azurants optiques, les laques, les pigments organiques, les pigments composites, et leurs mélanges.

L'invention porte sur une composition cosmétique pour application topique sur les matières kératiniques, en particulier la peau, sous forme d'émulsion comprenant, dans un milieu physiologiquement acceptable :
(i) au moins une dispersion d'oxychlorure de bismuth (Cl 77163) dans dans de l'hydroxystéarate d'éthyl (2) hexyle (nom INCl ethylhexyl hydroxystearate), et
(ii) au moins un agent soft focus, autrement nommé 'agent à effet de flou'
(iii) au moins une huile siliconée phénylée.

L'invention concerne également un procédé cosmétique de soin et/ou de maquillage des matières kératiniques comprenant l'application, sur lesdites matières kératiniques, en particulier sur la peau, d'une composition selon l'invention, par exemple sous la forme d'une couche de composition ou de plusieurs touches de composition appliquées sur la peau du visage, utilisée seule ou en association avec une autre composition (application double geste), ladite composition selon l'invention étant appliquée avant et/ou après l'application d'une composition de soin ou d'une composition de maquillage telle qu'un fond de teint.

En particulier, l'application de ladite composition sur la peau, notamment du visage, apporte à celle-ci un effet lumineux et de la couvrance.

De préférence, ladite matière colorante est présente dans la composition en une teneur allant de 0.001 à 3% en poids par rapport au poids total de ladite composition, en particulier de 0.05 à 2% en poids par rapport au poids total de ladite composition.

Selon un mode particulier et préféré, la composition selon l'invention est sous la forme d'une émulsion eau-dans-huile.

Selon un mode particulier, la composition selon l'invention est une composition de maquillage du visage, en particulier un fond de teint ou un enlumineur de teint.

Selon un mode particulier, ladite composition selon l'invention contient en outre au moins un ingrédient additionnel choisi parmi une charge, une matière colorante et/ou réfléchissante additionnelle, et leurs mélanges.

L'invention porte encore sur un procédé cosmétique de soin et/ou de maquillage des matières kératiniques comprenant l'application, sur lesdites matières kératiniques, en particulier sur la peau, d'une composition selon l'invention. En particulier, l'application de ladite composition sur la peau, notamment du visage, apporte à celle-ci un effet lumineux et un éclat de la peau avec avantageusement un effet bonne mine.

L'application sur les matières kératiniques et la peau du visage en particulier peut se faire par exemple sous la forme d'une couche de composition ou de plusieurs touches de composition appliquées sur la peau du visage, utilisée seule ou en association avec une autre composition (application double geste), ladite composition selon l'invention étant appliquée avant et/ou après l'application d'une composition de soin ou d'une composition de maquillage telle qu'un fond de teint.

En particulier, l'application de ladite composition sur la peau, notamment du visage, apporte à celle-ci un effet lumineux et de l'éclat et avantageusement un effet bonne mine.

Elle porte enfin sur l'association comprenant (i) au moins un premier pigment composite oxyde de titane/ Blue n° 1 Aluminium lake et un second pigment composite oxyde de titane/ / Red n° 28 Aluminium lake et D&C Red n° 7 et (ii) au moins de l'oxychlorure de bismuth dispersé dans au moins de l'hydroxystéarate d'éthyl (2) hexyle, susceptible d'être mise en oeuvre dans un procédé de préparation cosmétique ou une composition cosmétique ou un procédé de traitement cosmétique tels que définis dans l'une quelconque des revendications.

### DISPERSION D'OXYCHLORURE DE BISMUTH

L'oxychlorure de bismuth utilisé pour la préparation d'une composition selon l'invention est dispersé dans au moins une huile telle que définie ci-après (mélange liquide) et se distingue de l'oxychlorure de bismuth classique sous forme de poudre.

On parlera ainsi de 'dispersion' ou de 'pré-dispersion' d'oxychlorure de bismuth dans une huile pour définir selon l'invention la matière première mise en oeuvre dans un procédé de préparation d'une composition cosmétique selon l'invention.

On parlera d'oxychlorure de bismuth 'dispersé dans au moins une huile' telle que définie selon l'invention ou de 'mélange d'oxychlorure de bismuth avec ladite huile', pour définir la matière première une fois formulée dans la composition de l'invention.

La dispersion (autrement nommée 'pré-dispersion') d'oxychlorure de bismuth dans de l'hydroxystéarate d'éthyl (2) hexyle.

Selon un mode particulier et préféré, la composition selon l'invention comprend ainsi, dans un milieu physiologiquement acceptable,
a. au moins une phase aqueuse formant de 30 à 70% en poids par rapport au poids total de ladite composition et au moins 5% en poids par rapport au poids total de la composition d'au moins un monoalcool en C₂-C₈; et
b. au moins de l'oxychlorure de bismuth dispersé dans au moins une huile choisie parmi l'hydroxystearate d'éthyl (2) hexyle,
c. et au moins une huile choisie les huiles siliconées phénylées.

L'oxychlorure de bismuth sera généralement présent dans la dispersion (pré-dispersion) en présence du dispersant huileux (huile), en une teneur en poids allant de 50 à 90%, notamment de 60 à 80%, et mieux de 65 à 75% en poids par rapport au poids total de la dispersion. Le dispersant huileux sera par conséquent présent dans la dispersion en une teneur allant de 10 à 50% en poids, en particulier de 20 à 40% en poids, et mieux de 25 à 35% en poids.

Selon un mode particulier, on utilise une dispersion (pré-dispersion) comprenant de l'oxychlorure de bismuth et un dispersant huileux dans un rapport pondéral allant de 2 à 3.

Selon un mode préféré, on utilise une dispersion (pré-dispersion) comprenant 68% à 72% en poids d'oxychlorure de bismuth dans 28% à 32% en poids d'hydroxystéarate d'éthyl (2) hexyle, par rapport au poids total de la dispersion, soit un rapport pondéral oxychlorure de bismuth/dispersant huileux supérieur ou égal à 2, de préférence compris entre 2 et 2,6.

Une telle dispersion (pré-dispersion) est notamment commercialisée sous la dénomination Biron^{®} Liquid Silver par la société MERCK.

La dispersion (pré-dispersion) d'oxychlorure de bismuth sera utilisée en une teneur en matière première allant de 0,05 à 30% en poids, de préférence de 0,2 à 25%, de préférence encore de 0,5 à 20% en mieux de 1 à 15% en poids par rapport au poids total de ladite composition selon l'invention.
Une composition selon l'invention pourra ainsi comprendre de l'oxychlorure de bismuth en une teneur en matière active allant de 0,035 à 21% en poids, de préférence de 0,14 à 17,5% en poids, de préférence encore de 0,35 à 14% en poids, et mieux de 0,7 à 10,5% en poids de matière active par rapport au poids total de ladite composition avec un rapport pondéral oxychlorure de bismuth/ethyl hexyl hydroxystearate allant de 2 à 3, de préférence de 2 à 2,6.

Selon un mode particulier, la dispersion (pré-dispersion) d'oxychlorure de bismuth sera utilisée en une teneur en matière première allant de 0,01 à 15% en poids, de préférence de 0,5 à 10% en poids par rapport au poids total de ladite composition selon l'invention. Autrement dit, la teneur totale en oxychlorure de bismuth et en huile associée, telle que définie précédemment (dispersant huileux), ira de de 0,01 à 15% en poids, de préférence de 0,5 à 10% en poids par rapport au poids total de ladite composition selon l'invention, notamment avec un rapport pondéral oxychlorure de bismuth/dispersant huileux (huile associée) allant de 2 à 3.

Selon un mode particulier, ces valeurs ne tiennent pas compte des teneurs des éventuelles huiles additionnelles présentes dans la composition finale.

La teneur en matière première de la dispersion (pré-dispersion) d'oxychlorure de bismuth pourra être ajustée par l'homme du métier selon que la composition contient ou non en outre des matières colorantes.

A titre d'exemple, lorsque la composition selon l'invention n'est pas colorée et est par exemple utilisée comme base de soin et/ou de maquillage, la teneur en matière première de dispersion (pré-dispersion) d'oxychlorure de bismuth ira généralement de de 0,01 à 30%, notamment de 0,1 à 30%, en particulier de 1 à 30%, de préférence de 2 à 20%, mieux de 5 à 15% en poids de matière première par rapport au poids total de ladite composition selon l'invention.

Selon un mode préféré, lorsque la composition selon l'invention n'est pas colorée la teneur en matière première de dispersion (pré-dispersion) d'oxychlorure de bismuth, autrement dit la teneur totale en oxychlorure de bismuth et huile associée telle que définie précédemment, ira généralement de 0,01 à 15%, de préférence de 0,1 à 10% en poids par rapport au poids total de ladite composition selon l'invention, notamment avec un rapport pondéral oxychlorure de bismuth/dispersant huileux (huile associée) allant de 2 à 3.
Et lorsque la composition selon l'invention est colorée, c'est-à-dire qu'elle comprend en outre des matières colorantes (pigments, nacres, colorants), la teneur en matière première de dispersion (pré-dispersion) d'oxychlorure de bismuth ira généralement de 0,01 à 20%, notamment de 0,05 à 20%, de préférence de 0,2 à 15%, mieux de 0,5 à 10% en poids de matière première par rapport au poids total de ladite composition selon l'invention.

Selon un mode particulier, lorsque la composition selon l'invention est colorée, c'est-à-dire qu'elle comprend en outre des matières colorantes (pigments, nacres, colorants) par exemple en une teneur allant de 0,01 à 8% en poids par rapport au poids total de ladite composition, la teneur en matière première de dispersion (pré-dispersion) d'oxychlorure de bismuth, autrement dit la teneur totale en oxychlorure de bismuth et huile associée telle que définie précédemment, ira généralement de 0,05 à 12%, de préférence de 1 à 11% en poids de matière première par rapport au poids total de ladite composition selon l'invention. Pour une teneur plus importante en matières colorantes additionnelles, par exemple allant de 8 à 25% en poids par rapport au poids total de ladite composition, la teneur en matière première de dispersion (pré-dispersion) d'oxychlorure de bismuth, autrement dit la teneur totale en oxychlorure de bismuth et huile associée telle que définie précédemment, ira généralement de 1,2% à 15% en poids, de préférence de 1,5 à 12% en poids par rapport au poids total de ladite composition.

Selon un mode particulier, ces valeurs ne tiennent pas compte des teneurs des éventuelles huiles additionnelles présentes dans la composition finale.

La composition selon l'invention comprend en outre au moins une siliconée phénylée.

### Huiles siliconées phénylées

On entend par silicone phénylée (également appelée huile siliconée phénylée), un organopolysiloxane substitué par au moins un groupe phényle.

La silicone phénylée est de préférence non volatile. Par « non volatile », on entend une huile dont la pression de vapeur à température ambiante et pression atmosphérique, est non nulle et inférieure à 0,02 mm de Hg (2,66 Pa) et mieux inférieure à 10⁻³ mm de Hg (0,13 Pa).

L'huile siliconée phénylée peut être choisie parmi les phényl triméthicones, les phényl diméthicones, les phényl triméthylsiloxy diphénylsiloxanes, les diphényl diméthicones, les diphényl méthyldiphényl trisiloxanes, le triméthyl pentaphényl trisiloxane , les 2-phényléthyl triméthylsiloxysilicates.

L'huile siliconée peut répondre à la formule: 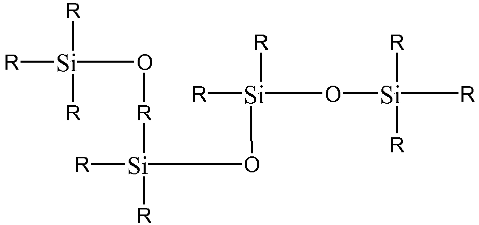 dans laquelle les groupements R représentent indépendamment les uns des autres un méthyle ou un phényle. De préférence dans cette formule, l'huile siliconée comprend au moins trois groupes phényle, par exemple au moins quatre, au moins cinq ou au moins six.

Selon un autre mode de mise en oeuvre, l'huile siliconée répond à la formule 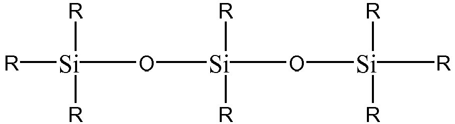 dans laquelle les groupements R représentent indépendamment les uns des autres un méthyle ou un phényle. De préférence dans cette formule, ledit organopolysiloxane comprend au moins trois groupes phenyls, par exemple au moins quatre ou au moins cinq.

Des mélanges des organopolysiloxanes phénylés décrits précédemment peuvent être utilisés.

On peut citer par exemples des mélanges d'organopolysiloxane triphénylé, tétra- ou penta-phénylé.

Selon un autre mode de mise en oeuvre, l'huile siliconée répond à la formule 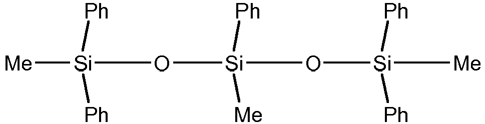 dans laquelle Me représente méthyle, Ph représente phényle. Une telle silicone phénylée est notamment fabriquée par Dow Corning sous la référence Dow Corning 555 Cosmetic Fluid (nom INCI : trimethyl pentaphenyl trisiloxane). La référence Dow Corning 554 Cosmetic Fluid peut aussi être utilisée.

Selon un autre mode de mise en oeuvre, l'huile siliconée répond à la formule 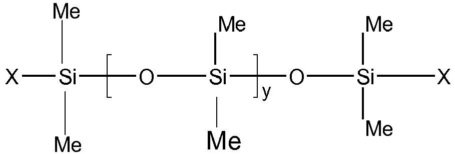 dans laquelle Me représente méthyle, y est compris entre 1 et 1 000, et X représente -CH₂-CH(CH₃)(Ph).

Selon un autre mode de mise en oeuvre, l'huile siliconée répond à la formule 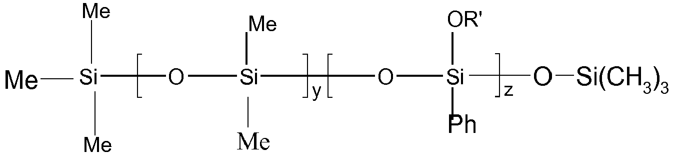 dans laquelle -OR' représente -O-SiMe3, y est compris entre 1 et 1 000 et z est compris entre 1 et 1 000.

L'huile siliconée phénylée peut être choisie parmi les silicones phénylées de formule (VI) suivante : 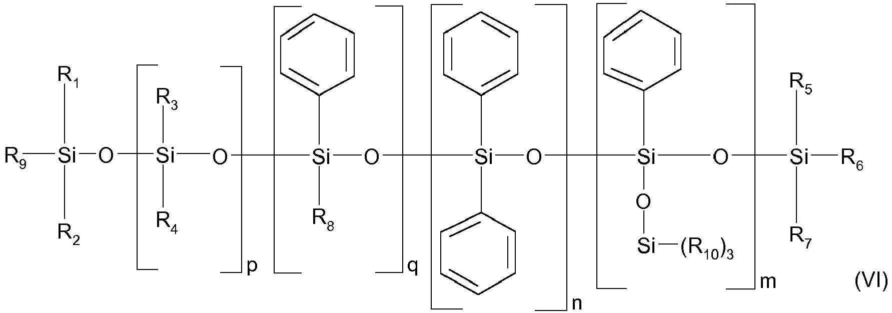 dans laquelle
- R1 à R10, indépendamment les uns des autres, sont des radicaux hydrocarbonés, saturés ou insaturés, linéaires, cycliques ou ramifiés, en C1-C30,
- m, n, p et q sont, indépendamment les uns des autres, des nombres entiers compris entre 0 et 900, sous réserve que la somme 'm+n+q' est différente de 0.
De préférence, la somme 'm+n+q' est comprise entre 1 et 100. De préférence, la somme 'm+n+p+q' est comprise entre 1 et 900, encore mieux entre 1 et 800. De préférence, q est égal à 0.

L'huile siliconée phénylée peut être choisie parmi les silicones phénylées de formule (VII) suivante : 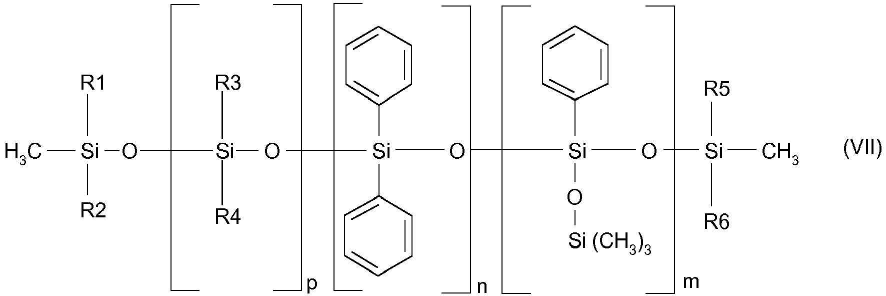 dans laquelle :
- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m' est comprise entre 1 et 100.

De préférence, R1 à R6, indépendamment les uns des autres, représentent un radical hydrocarboné saturé, linéaire ou ramifié, en C1-C30, notamment en C1-C12, et en particulier un radical méthyle, éthyle, propyle ou butyle.
Notamment, R1 à R6 peuvent être identiques, et en outre peuvent être un radical méthyle.

De préférence, on peut avoir m=1 ou 2 ou 3, et/ou n=0 et/ou p=0 ou 1., dans la formule (VII).

On peut utiliser une huile siliconée phénylée de formule (VI) ayant une viscosité à 25°C comprise entre 5 et 1500 mm²/s (soit 5 à 1500 cSt), de préférence ayant une viscosité comprise entre 5 et 1000 mm²/s (soit 5 à 1000 cSt).

Comme huile siliconée phénylée de formule (VII), on peut utiliser notamment les phényltriméthicones telles que la DC556 de Dow Corning (22,5 cSt), l'huile Silbione 70663V30 de Rhône Poulenc (28 cSt), ou les diphényldiméthicones telles que les huiles Belsil, notamment Belsil PDM1000 (1000cSt), Belsil PDM 200 (200 cSt) et Belsil PDM 20 (20cSt) de Wacker. Les valeurs entre parenthèses représentent les viscosités à 25°C.

L'huile siliconée non volatile peut être choisie parmi les silicones de formule : 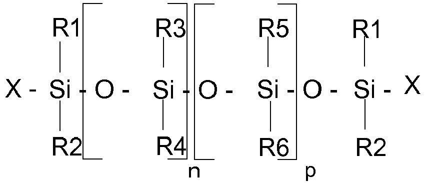 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à l'huile une masse moléculaire en poids inférieure à 200 000 g/mol , de préférence inférieure à 150 000 g/mol et de préférence encore inférieure à 100 000 g/mol.

L'indice de réfraction de l'huile silicone phénylée sera de préférence > à 1,42. De préférence l'indice sera avantagesement supérieur à 1,43, de préférence supérieur à 1,45 et de préférence encore supérieur à 1,46.

L'huile hydrocarbonée d'indice de réfraction > à 1,42 et de δₐ >1 et /ou l'huile de silicone phénylée selon l'invention est présente dans la composition en une teneur allant de 0, 1 à 50% en poids par rapport au poids total de ladite composition, de préférence de 0,5 à 30% en poids, et mieux de 1 à 20% en poids par rapport au poids total de la composition.

### Galénique

La présente invention concerne des compositions cosmétiques notamment de type émulsions pouvant se présenter sous la forme d'une composition de soin et/ou de maquillage des matières kératiniques, notamment de la peau, ou encore sous la forme d'une composition de protection solaire.

Elle peut se présenter alors sous une forme non colorée, contenant éventuellement des actifs cosmétiques ou dermatologiques. Elle peut alors être utilisée comme base de soin ou de maquillage pour les matières kératiniques, notamment la peau.

Une composition de l'invention peut également se présenter sous forme d'un produit coloré de maquillage des matières kératiniques, notamment pour le maquillage de la peau, tel qu'un fond de teint notamment à appliquer sur le visage ou le cou, un produit anti-cernes, un correcteur de teint, une crème teintée, une composition de maquillage pour le corps.

La composition selon l'invention peut se présenter sous diverses formes, notamment sous forme de composition anhydre, de dispersion, émulsion, telle que notamment eau/huile ou huile/eau ou multiples.

Une composition de l'invention est de préférence une émulsion, en particulier directe ou inverse, ou une composition anhydre.

Une dispersion peut être effectuée en phase aqueuse ou en phase huileuse.

Une émulsion peut posséder une phase continue huileuse ou aqueuse. Une telle émulsion peut être, par exemple, une émulsion inverse (E/H) ou directe (H/E), ou encore une émulsion multiple (E/H/E ou H/E/H).

Dans le cas des émulsions, les émulsions inverses (E/H) sont préférentielles.

Une composition anhydre est une composition contenant moins de 5 % en poids d'eau, voire moins de 2 % d'eau, et notamment exempte d'eau. Le cas échéant, d'aussi faibles quantités d'eau peuvent notamment être amenées par des ingrédients de la composition qui peuvent en contenir des quantités résiduelles.

En particulier, la composition selon l'invention peut être est sous la forme d'une émulsion huile-dans-eau (H/E), d'une émulsion eau-dans-huile (E/H) ou d'une émulsion multiple, de préférence une émulsion eau-dans-huile (E/H).

La composition selon l'invention peut constituer une composition de base, ou une composition de soin et/ou de maquillage, ou une composition à appliquer sous ou sur une autre composition de maquillage et/ou de soin, par petites touches pour unifier le teint.

Dans le cas où la composition selon l'invention est une base de soin et/ou de maquillage, elle pourra être avantageusement non pigmentée, permettant un usage dans ladite base d'une forte teneur en dispersion (pré-dispersion) d'oxychlorure de bismuth dans hydroxystearate d'ethyl (2) hexyle. Cette base de soin et/ou de maquillage peut être appliquée sur l'ensemble du visage, avant l'application d'une composition de soin et/ou d'une composition de maquillage. Selon un autre mode, ladite base de soin et/ou de maquillage pourra s'utiliser en touches de maquillage, avant (dessous) ou après (dessus) l'application d'une couche d'une composition de soin ou de maquillage (fond de teint).

Selon un mode particulier, ladite composition est appliquée par touches sur les zones du visage présentant des défauts cutanés ; cette application peut se faire avant l'application subséquente d'un fond de teint ou après. Selon un autre mode particulier, elle peut être appliquée autour des yeux pour illuminer le regard ou au-dessus des lèvres, sur l'arc de cupidon, pour donner du galbe.

Selon un mode particulier, ladite composition est appliquée sur des personnes à peau présentant des irrégularités visibles et/ou tactiles et/ou une hétérogénéité du teint.

Selon un mode particulier de l'invention, ladite composition est appliquée sur des personnes à peau grasse.

Selon un autre mode particulier de l'invention, ladite composition est appliquée sur des personnes à peau âgée ou à peau mature.

Selon un autre mode particulier de l'invention, ladite composition est appliquée sur des personnes à peau asiatique.

### MILIEU PHYSIOLOGIQUEMENT ACCEPTABLE

Outre, les composés indiqués précédemment, une composition selon l'invention comprend un milieu physiologiquement acceptable.

Par « milieu physiologiquement acceptable », on entend désigner un milieu convenant particulièrement à l'application d'une composition de l'invention sur les matières kératiniques, notamment la peau.

Le milieu physiologiquement acceptable est généralement adapté à la nature du support sur lequel doit être appliquée la composition, ainsi qu'à l'aspect sous lequel la composition doit être conditionnée.

### Phase aqueuse

Une composition de l'invention peut comprendre une phase aqueuse en une teneur variant de 0 à 80%, en particulier de 1 à 80% en poids, notamment de 10 à 80 %, et plus particulièrement de 20 à 60 % en poids, notamment de 30 à 50% en poids par rapport au poids total de la composition.

Selon un mode particulier, une composition de l'invention comprend une phase aqueuse en une teneur variant de 10 à 80 %, et plus particulièrement de 20 à 60 % en poids, notamment de 30 à 50% en poids par rapport au poids total de la composition.

La phase aqueuse comprend généralement de l'eau telle que : une eau déminéralisée, une eau de source naturelle, telle que l'eau de La Roche-Posay, une eau florale telle que l'eau de bleuet et/ou une eau thermale.

Selon un mode de réalisation, une composition de l'invention peut comprendre en outre au moins un solvant organique miscible à l'eau.

Le ou les solvant(s) organique(s) miscible(s) à l'eau convenant à l'invention peu(vent)t être choisi(s) parmi les monoalcools en C₁₋₈, et notamment en C₁₋₅, notamment l'éthanol, l'isopropanol, le tert-butanol, le n-butanol, les polyols tels que décrits précédemment, et leurs mélanges.

Une composition de l'invention peut en outre comprendre au moins un sel, par exemple, le chlorure de sodium, le chlorure de magnésium et le sulfate de magnésium.

Une composition de l'invention peut comprendre de 0,05 % à 1,5 % en particulier de 0,1 % à 1,0 % et plus particulièrement de 0,15 % à 0,8 % en poids de sels, par rapport au poids total de la composition.
Selon un mode particulier, la phase aqueuse forme au moins 30 % en poids, en particulier au moins 45 % en poids, voire plus de 50 % en poids et notamment plus de 60 % en poids du poids total de la composition selon l'invention. De préférence, la phase aqueuse est présente dans l'émulsion selon l'invention en une teneur allant 30 % à 70 %, en poids, de préférence allant de 40 à 60% en poids, et mieux de 50 % à 60 % en poids, par rapport au poids total de l'émulsion.

La composition peut comprendre en outre, en particulier lorsque la composition comprend une phase aqueuse représentant au moins 30% en poids par rapport au poids total de la composition, au moins un mono-alcool inférieur en C₂ à C₈-

Les mono-alcools inférieurs convenant plus particulièrement à l'invention comportent de 2 à 8 atomes de carbone, notamment de 2 à 6 atomes de carbone, et en particulier de 2 à 4 atomes de carbone, comme l'éthanol, l'isopropanol, le propanol, le butanol.

Convient aussi tout particulièrement à l'invention, l'éthanol et l'isopropanol et de préférence l'éthanol.

Comme précisé précédemment, cet alcool est présent à raison d'au moins 5 %, en particulier d'au moins 7 %, notamment à raison d'au moins 10 % en poids par rapport au poids total de la composition.
Pour des raisons de confort, on privilégiera une teneur inférieure ou égale à 20 % en poids et notamment inférieure ou égale à 15 % en poids de mono-alcool par rapport au poids total des compositions selon l'invention, autrement dit une teneur inférieure ou égale à 40% en poids et notamment inférieure ou égale à 30% en poids par rapport au poids total de la phase aqueuse desdites compositions.

Ainsi, le mono-alcool peut représenter au moins 10 % en poids, voire 20 % en poids et jusqu'à 40 % en poids de la phase aqueuse.

### Phase grasse liquide

Une composition cosmétique conforme à la présente invention peut comprendre au moins une phase grasse liquide et/ou solide et notamment au moins une huile comme mentionnée ci-après.

On entend par huile, tout corps gras sous forme liquide à température ambiante (20 - 25 °C) et à pression atmosphérique.

Une composition de l'invention peut comprendre une phase grasse liquide en une teneur variant de 5 à 95 %, en particulier de 10 à 80 %, en particulier de 15 à 70 %, et plus particulièrement, de 20 à 65 % en poids par rapport au poids total de la compositi n.

La phase huileuse convenant à la préparation des compositions cosmétiques selon l'invention peut comprendre des huiles hydrocarbonées, siliconées, fluorées ou non, ou leurs mélanges.

Les huiles peuvent être volatiles ou non volatiles.

Elles peuvent être d'origine animale, végétale, minérale ou synthétique.

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et à pression atmosphérique, en particulier, ayant une pression de vapeur allant de 0,13 Pa à 40 000 Pa (10⁻³ à 300 mm Hg), et de préférence, allant de 1,3 Pa à 13 000 Pa (0,01 à 100 mm Hg), et préférentiellement allant de 1,3 Pa à 1300 Pa (0,01 à 10 mm Hg).

Au sens de la présente invention, on entend par « huile non volatile », une huile ayant une pression de vapeur inférieure à 0,13 Pa.

Au sens de la présente invention, on entend par « huile siliconée », une huile comprenant au moins un atome de silicium, et notamment au moins un groupe Si-O.

On entend par « huile fluorée », une huile comprenant au moins un atome de fluor.

On entend par « huile hydrocarbonée », une huile contenant principalement des atomes d'hydrogène et de carbone.

Les huiles peuvent éventuellement comprendre des atomes d'oxygène, d'azote, de soufre et/ou de phosphore, par exemple, sous la forme de radicaux hydroxyle ou acide.

### Huiles volatiles

Les huiles volatiles peuvent être choisies parmi les huiles hydrocarbonées ayant de 8 à 16 atomes de carbone, et notamment les alcanes ramifiés en C₈-C₁₆ (appelées aussi isoparaffines), comme l'isododécane (encore appelé 2,2,4,4,6-pentaméthylheptane), l'isodécane, l'isohexadécane, et, par exemple, les huiles vendues sous les noms commerciaux d'ISOPARS^{®} ou de PERMETHYLS^{®}, les alcanes linéaires volatils, et leurs mélanges.

Selon un mode de réalisation avantageux, les alcanes linéaires volatils convenant à l'invention présentent une vitesse d'évaporation allant de 0,01 à 3,5 mg/cm²/min, à température ambiante (25°C) et pression atmosphérique (760 mm Hg), et comprennent de 9 à 14 atomes de carbone. A titre d'exemple, on peut citer le le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges. De préférence, le ou les alcanes linéaires volatils sont choisis parmi le n-undécane, le n-dodécane, le n-tridécane, le n-tétradécane, et leurs mélanges. Selon un mode préféré, la composition selon l'invention comprend du dodécane. Selon un autre mode préféré, la composition selon l'invention comprend du tétradécane. Selon un autre mode préféré, on peut citer les mélanges de n-undécane (C11) et de n-tridécane (C13) obtenus aux exemples 1 et 2 de la demande WO2008/155059 de la Société Cognis. On peut également citer le n-dodécane (C12) et le n-tétradécane (C14) tels que ceux vendus par Sasol respectivement sous les références PARAFOL 12-97 et PARAFOL 14-97, ainsi que leurs mélanges.

Comme huiles volatiles, on peut aussi utiliser les silicones volatiles, comme, par exemple, les huiles de silicones linéaires ou cycliques volatiles, notamment, celles ayant une viscosité ≤ 8 centistokes (cSt) (8 x 10⁻⁶ m²/s), et ayant, notamment, de 2 à 10 atomes de silicium, et en particulier, de 2 à 7 atomes de silicium, ces silicones comportant, éventuellement, des groupes alkyle ou alkoxy ayant de 1 à 10 atomes de carbone. Comme huile de silicone volatile utilisable dans l'invention, on peut citer, notamment, les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées, telles que le nonafluorométhoxybutane ou le perfluorométhylcyclopentane, et leurs mélanges.

### Huiles non volatiles

Les huiles non volatiles peuvent, notamment, être choisies parmi les huiles hydrocarbonées, fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale, telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier, dont les acides gras peuvent avoir des longueurs de chaînes variant de C₄ à C₃₆, et, notamment, de C₁₈ à C₃₆, ces huiles pouvant être linéaires ou ramifiées, saturées ou insaturées ; ces huiles peuvent, notamment, être des triglycérides héptanoïques ou octanoïques, l'huile de karité, de luzerne, de pavot, de millet, d'orge, de seigle, de bancoulier, de passiflore, le beurre de karité, l'huile d'aloès, l'huile d'amande douce, l'huile d'amande de pêche, l'huile d'arachide, l'huile d'argan, l'huile d'avocat, l'huile de baobab, l'huile de bourrache, l'huile de brocoli, l'huile de calendula, l'huile de caméline, l'huile de canola, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de lys, l'huile de macadamia, l'huile de maïs, l'huile de meadowfoam, l'huile de millepertuis, l'huile de monoï, l'huile de noisette, l'huile de noyaux d'abricot, l'huile de noix, l'huile d'olive, l'huile d'onagre, l'huile de palme, l'huile de pépins de cassis, l'huile de pépins de kiwi, l'huile de pépins de raisin, l'huile de pistache, l'huile de potimarron, l'huile de potiron, l'huile de quinoa, l'huile de rosier muscat, l'huile de sésame, l'huile de soja, l'huile de tournesol, l'huile de ricin, et l'huile de watermelon, et leurs mélanges, ou encore des triglycérides d'acides caprylique/caprique, comme ceux vendus par la société STEARINERIES DUBOIS ou ceux vendus sous les dénominations MIGLYOL 810^{®}, 812^{®} et 818^{®} par la société DYNAMIT NOBEL,
- les éthers de synthèse ayant de 10 à 40 atomes de carbone, comme le dicapryl ether,
- les esters de synthèse, comme les huiles de formule R₁COOR₂, dans laquelle R₁ représente un reste d'un acide gras linéaire ou ramifié comportant de 1 à 40 atomes de carbone et R₂ représente une chaîne hydrocarbonée, notamment, ramifiée contenant de 1 à 40 atomes de carbone à condition que R₁ + R₂ soit ≥ 10. Les esters peuvent être, notamment, choisis parmi les esters d'alcool et d'acide gras, comme par exemple, l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate d'isopropyle, le stéarate d'octyle, les esters hydroxylés, comme le lactate d'isostéaryle, l'hydroxystéarate d'octyle, les ricinoléates d'alcools ou de polyalcools, le laurate d'hexyle, les esters de l'acide néopentanoïque, comme le néopentanoate d'isodécyle, le néopentanoate d'isotridécyle, les esters de l'acide isononanoïque, comme l'isononanoate d'isononyle, l'isononanoate d'isotridécyle,
- les esters de polyols et les esters du pentaérythritol, comme le tétrahydroxystéarate/tétraisostéarate de dipentaérythritol,
   les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique,
- les acides gras supérieurs en C₁₂-C₂₂, tels que l'acide oléique, l'acide linoléique, l'acide linolénique, et leurs mélanges,
- les carbonates de di-alkyle, les 2 chaînes alkyles pouvant être identiques ou différentes, tels que le dicaprylyl carbonate commercialisé sous la dénomination CETIOL CC^{®}, par COGNIS, et
- les huiles de masse molaire élevée ayant, en particulier, une masse molaire allant d'environ 400 à environ 2000 g/mol, en particulier, d'environ 650 à environ 1600 g/mol. Comme huile de masse molaire élevée utilisable dans la présente invention, on peut notamment citer les esters d'acides gras linéaires ayant un nombre total de carbone allant de 35 à 70, comme le tétrapélargonate de pentaérythrityle, les esters hydroxylés, tels que le triisostéarate de polyglycérol-2, les esters aromatiques, tels que le tridécyl trimellitate, les esters d'alcools gras ou d'acides gras ramifiés en C₂₄-C₂₈, tels que ceux décrits dans le brevet US 6,491,927 et les esters du pentaérythritol, et, notamment, le citrate de triisoarachidyle, le triisostéarate de glycéryle, le tri décyl-2 tétradécanoate de glycéryle, le tétraisostéarate de polyglycéryle-2 ou encore le tétra décyl-2 tétradécanoate de pentaérythrityle ; les silicones phénylées comme la BELSIL PDM 1000 de la société WACKER (MM=9000 g/mol), les polydiméthylsiloxanes (PDMS) non volatiles, les PDMS comportant des groupements alkyle ou alcoxy pendants et/ou en bouts de chaîne siliconée, groupements ayant chacun de 2 à 24 atomes de carbone, les silicones phénylées, comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, et leurs mélanges ; ainsi que les mélanges de ces différentes huiles.

### Agents Tensioactifs

Une composition selon l'invention comprend généralement au moins un agent tensioactif, notamment choisi parmi les agents tensioactifs amphotères, anioniques, cationiques ou non ioniques, utilisés seuls ou en mélange.

Les agents tensioactifs sont généralement présents dans la composition, en une proportion pouvant aller par exemple de 0,3 à 20 % en poids, en particulier de 0,5 à 15 % en poids, et plus particulièrement de 1 à 10 % en poids d'agents tensioactifs par rapport au poids total de la composition.

Bien entendu, l'agent tensioactif est choisi de manière à stabiliser efficacement les émulsions plus particulièrement considérées selon l'invention à savoir de type H/E, E/H, ou H/E/H. Ce choix relève des compétences de l'homme de l'art.

Pour les émulsions H/E, on peut citer par exemple les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) (nom CTFA : glycéryl stéarate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stéarate), le monostéarate de polyéthylène glycol 100 OE (nom CTFA: PEG-100 stéarate) et leurs mélanges.

On peut aussi utiliser des mélanges de ces agents tensioactifs, comme par exemple le produit contenant du Glycéryl stéarate et du PEG-100 stéarate, commercialisé sous la dénomination ARLACEL 165 par la société Uniqema, et le produit contenant du Glycéryl stéarate (mono-distéarate de glycéryle) et du stéarate de potassium, commercialisé sous la dénomination TEGIN par la société Goldschmidt (nom CTFA : glyceryl stearate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Méthyl glucose dioleate/hydroxystearate) ; l'ester de méthylglucoside et d'acide isostéarique (nom CTFA : Méthyl glucose isostearate) ; l'ester de méthylglucoside et d'acide laurique (nom CTFA : Méthyl glucose laurate) ; le mélange de monoester et de diester de méthylglucoside et d'acide isostéarique (nom CTFA : Methyl glucose sesqui-isostéarate) ; le mélange de monoester et de diester de méthylglucoside et d'acide stéarique (nom CTFA : Méthyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distéarate) tel que le produit commercialisé sous la dénomination Glucam E-20 distéarate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 méthyl glucose sesquistéarate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges. Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à 22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le décylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic, et leurs mélanges.

Pour les émulsions E/H, on peut utiliser des agents tensioactifs hydrocarbonés ou siliconés.

Selon une variante de réalisation, les tensioactifs hydrocarbonés sont préférés.

On peut citer par exemple comme agents tensioactifs hydrocarbonés, les polyesters de polyols comme le PEG-30 dipolyhydroxystearate vendu sous la référence ARLACEL P 135 par la société Uniqema, le polyglyceryl-2 dipolyhydroxystearate vendu sous la référence DEHYMULS PGPH par la société Cognis.

On peut citer par exemple comme agents tensioactifs siliconés les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt.

On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple le polyglyceryl-3 diisostearate commercialisé sous la dénomination de LAMEFORM TGI par la société Cognis, l'isostéarate de polyglycérol-4, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

On peut aussi utiliser comme agents tensioactifs d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Conviennent également à l'invention, les agents tensioactifs de type polymères amphiphiles.

On entend par polymère amphiphile, tous polymères comportant à la fois une partie hydrophile et une partie hydrophobe et ayant la propriété de former un film séparant deux liquides de polarité différente et permettant ainsi de stabiliser des dispersions liquide - liquide de type direct, inverse ou multiple. Ces polymèrespeuvent être hydrosolubles ou hydrodispersibles.

On peut utiliser plus particulièrement les copolymères acrylate/C₁₀-C₃₀-alkylacrylate tels que les produits vendus sous les noms PEMULEN TR1, PEMULEN TR2 et CARBOBOL 1382 par la Société GOODRICH, ou bien leurs mélanges. On peut utiliser aussi les copolymères acrylate/steareth-20 itaconate et copolymères acrylate/ceteth-20 itaconate vendus sous les noms STRUCTURE 2001 et STRUCTURE 3001 par la Société NATIONAL STARCH. A titre de terpolymères pouvant être utilisés, on peut citer le terpolymère acide méthacrylique/acrylate de méthyle/diméthyl m-isopropénylbenzylisocyanate d'alcool béhénylique éthoxylé à 40 OE,(c'est-à-dire comportant 40 groupes oxyéthylénés) vendus par la société AMERCHOL sous les dénominations VISCOPHOBE DB 1000 NP3-NP4.

On peut également citer les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous la dénomination SALCARE SC 80.

On peut également citer les polymères anioniques comme par exemple les polymères d'acide isophtalique ou d'acide sulfoisophtalique, et en particulier les copolymères de phtalate/sulfoisophtalate/glycol (par exemple diéthylèneglycol/Phtalate/isophtalate/1,4-cyclohexane-diméthanol) vendus sous les dénominations « Eastman AQ polymer » (AQ35S, AQ38S, AQ55S, AQ48 Ultra) par la société Eastman Chemical.

Selon un mode particulier, on utilise un agent tensioactif siliconé, en particulier choisi parmi les dimethicone copolyols.

### Diméthicone copolyol

Le diméthicone copolyol selon l'invention est un polydiméthyl méthyl siloxane oxypropyléné et/ou oxyéthyléné.

On peut utiliser comme diméthicone copolyol ceux répondant à la formule (II) suivante : 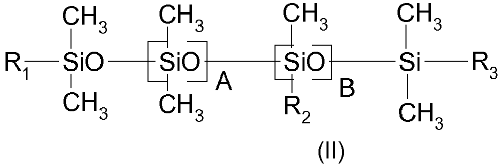 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)₂ - OR₄, au moins un radical R₁, R₂ ou R₃ n'étant pas un radical alkyle ; R₄ étant un hydrogène, un radical alkyle en C₁-C₃ ou un radical acyle en C₂-C₄ ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (II), R₁ = R₃ = radical méthyle, x est un nombre entier allant de 2 à 6 et y est un nombre entier allant de 4 à 30. R₄ est en particulier un hydrogène.

On peut citer, à titre d'exemples composés de formule (II), les composés de formule (III) : 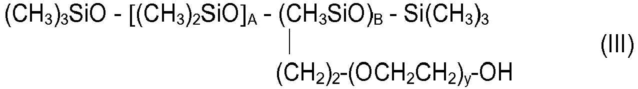 dans laquelle A est un nombre entier allant de 20 à 105, B est un nombre entier allant de 2 à 10 et y est un nombre entier allant de 10 à 20.

On peut également citer à titre d'exemple de composés siliconés de formule (II), les composés de formule (IV) :

HO - (OCH₂CH₂)_{y}-(CH₂)₃ - [(CH₃)₂SiO]_{A'} - (CH₂)₃ - (OCH₂CH₂)_{y} - OH (IV)

dans laquelle A' et y sont des nombres entiers allant de 10 à 20.

On peut utiliser comme diméthicone copolyol ceux vendus sous les dénominations DC 5329, DC 7439-146, DC 2-5695, Q4-3667 par la société Dow Corning ; KF-6013, KF-6015, KF-6016, KF-6017, KF-6028 par la société Shin-Etsu.
Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.

Le diméthicone copolyol peut être présent dans l'émulsion selon l'invention en une teneur allant de 1 % à 6 % en poids, par rapport au poids total de l'émulsion, et de préférence allant de 1,5 % à 4 % en poids, et préférentiellement allant de 2 % à 3 % en poids.

Selon une variante de réalisation préférée de l'invention, le diméthicone copolyol précité peut être associé à au moins une silicone oxyalkylénée substituée en α-ω.

### Silicone oxyalkylénée substituée en α-ω

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁₋C₁₀ et en particulier méthyle, les radicaux fluoroalkyles, les radicaux aryles et en particulier phényle. Ils peuvent être par exemple substitués par des groupements ester ou éther en C₁-C₄₀, des groupements aralkyles en C₇-C₆₀-

Ainsi, la silicone oxyalkyléne substituée en α-ω utilisable selon l'invention est un polymère organosilicié tel que défini ci-dessus, à structure linéaire, substitué aux deux extrémités de la chaîne principale par des groupements oxyalkylène reliés aux atomes de Si par l'intermédiaire d'un groupement hydrocarboné.

De préférence, la silicone oxyalkylénée substituée en α-ω répond à la formule générale (I) suivante : 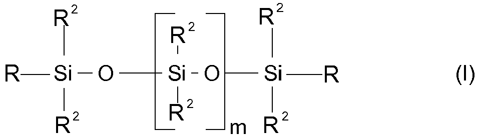 dans laquelle : R = -(CH₂)ₚ O-(C₂H₄O)ₓ (C₃H₆O)_{y}R¹
où :
- R¹ représente H, CH₃ ou CH₂CH₃,
- p est un entier allant de 1 à 5, x varie de 1 à 100, y varie de 0 à 50,
- les unités (C₂H₄O) et (C₃H₆O) pouvant être réparties de façon aléatoire ou
- par blocs,
- les radicaux R² représentent un radical alkyle en C₁-C₃ ou un radical phényle,
- 5 ≤ m ≤ 300,

De préférence, la silicone oxyalkylénée substituée en α-ω utilisée selon la présente invention répond à la formule générale (I) pour laquelle tous les radicaux R² sont des radicaux méthyles et :
- p va de 2 à 4,
- x va de 3 à 100,
- m va de 50 à 200.

De préférence encore, le poids moléculaire moyen de R va de 800 à 2600.

De préférence, le rapport en poids des unités C₂H₄O par rapport aux unités C₃H₆O va de 100:10 à 20:80. De façon avantageuse, ce rapport est d'environ 42/58.

De préférence encore, R¹ est le groupe méthyle.

De façon plus préférentielle encore, l'émulsion selon l'invention comprend la silicone oxyalkylénée en α-ω de formule suivante : 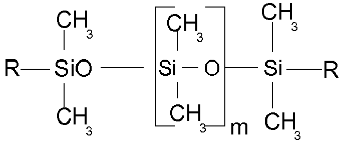 dans laquelle :
- m=100,
- R = (CH₂)₃-O-(C₂H₄O)ₓ-(C₃H₆O)_{y}-CH₃, où x va de 3 à 100, y va de 1 à 50, le rapport en poids du nombre de C₂H₄O sur le nombre de C₃H₆O étant d'environ 42/58, le poids moléculaire moyen de R allant de 800 à 1000.

La silicone oxyalkylénée substituée en α-ω telle que définie ci-dessus peut être utilisée selon l'invention en une proportion allant de 0,5 à 5 % en particulier de 1 à 4 % en poids et plus particulièrement de 2 à 3 % en poids par rapport au poids total de la composition.

Parmi les produits du commerce pouvant contenir tout ou partie des silicones oxyalkylénées substituées en α-ω utilisables selon l'invention comme émulsionnant, on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.

En particulier, il peut s'agir du cétyl diméthicone copolyol.

Cet agent tensioactif siliconé est avantageusement présent en une teneur allant de 0,5 à 5% en poids par rapport au poids total de ladite composition.

### GELIFIANTS

Selon la fluidité de la composition que l'on souhaite obtenir, on peut incorporer dans une composition de l'invention, un ou plusieurs gélifiants.

Un agent gélifiant convenant à l'invention peut être hydrophile, c'est-à-dire soluble ou dispersible dans l'eau.

Ceux-ci peuvent notamment être choisis parmi : les polymères carboxyvinyliques modifiés ou non, tels que les Carbopol (nom CTFA : carbomer) vendus par la société Goodrich ; les polyacrylates et polyméthacrylates tels que les produits vendus sous les dénominations de Lubrajel et Norgel par la société GUARDIAN ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide/C₁₃₋₁₄ Isoparaffin/Laureth-7) et sous le nom de SIMULGEL 600 (nom C.T.F.A. : Acrylamide/Sodium acryloyldimethyltaurate copolymer/Isohexadecane/Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose et l'hydroxypropylcellulose ; et leurs mélanges.

Un agent gélifiant convenant à l'invention peut être lipophile. Un gélifiant lipophile peut être minéral ou organique.

Comme gélifiants lipophiles, on peut citer par exemple les argiles modifiées telles que le silicate de magnésium modifié (Bentone gel VS38 de RHEOX), l'hectorite modifiée par le chlorure de distéaryl diméthyl ammonium (nom CTFA : Disteardimonium hectorite) commercialisé sous la dénomination « Bentone 38 CE » par la société RHEOX.

Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium telle que, par exemple, celle commercialisée sous la dénomination de Bentone 38V^{®} par la société ELEMENTIS.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6^{®}, KSG16^{®} et de KSG18^{®} par la société SHIN-ETSU, de Trefil E-505C^{®} et Trefil E-506C^{®} par la société DOW-CORNING, de Gransil SR-CYC^{®}, SR DMF10^{®}, SR-DC556^{®}, SR 5CYC gel^{®}, SR DMF 10 gel^{®} et de SR DC 556 gel^{®} par la société GRANT INDUSTRIES, de SF 1204^{®} et de JK 113^{®} par la société GENERAL ELECTRIC ; les copolymères séquencés de type « dibloc », « tribloc » ou « radial » du type polystyrène/polyisoprène, polystyrène/polybutadiène tels que ceux commercialisés sous la dénomination Luvitol HSB^{®} par la société BASF, du type polystyrène/copoly(éthylène-propylène) tels que ceux commercialisés sous la dénomination de Kraton^{®} par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans une composition cosmétique de l'invention, on peut encore citer les esters de dextrine et d'acide gras, tels que les palmitates de dextrine, notamment tels que ceux commercialisés sous les dénominations Rheopearl TL^{®}, Rheopearl TL2-OR^{®} ou Rheopearl KL^{®} par la société CHIBA FLOUR.

A titre de gélifiant lipophile convenant à l'invention, on peut également mentionner les huiles végétales hydrogénées, telles que l'huile de ricin hydrogénée.

A titre de gélifiant lipophile convenant également à l'invention, on peut mentionner les alcools gras, en particulier de C₈ à C₂₆, et plus particulièrement de C₁₂ à C₂₂.

Selon un mode de réalisation, un alcool gras convenant à l'invention peut être choisi parmi l'alcool mysritylique, l'alcool cétylique, l'alcool stéarylique, l'alcool béhénylique.

A titre de gélifiant lipophile convenant également à l'invention, on peut mentionner les esters d'acide gras et de glycérols, tels que le stéarate de glycéryle.

Selon un mode de réalisation, une composition de l'invention peut comprendre au moins un agent gélifiant lipophile, notamment choisi parmi les hectorites modifiées.

### MATIERES COLORANTES

Une composition selon l'invention peut en outre comprendre au moins une matière colorante, en particulier une matière colorante pulvérulente.

Une composition cosmétique conforme à l'invention peut, avantageusement, incorporer au moins une matière colorante choisie parmi des matières colorantes organiques ou inorganiques, notamment de type pigments ou nacres classiquement utilisés dans les compositions cosmétiques, liposolubles ou hydrosolubles, et leurs mélanges.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,01 à 40 % en poids, notamment, de 0,1 à 20 % en poids, et en particulier, de 1 à 15 % en poids, par rapport au poids total de la composition cosmétique.

Comme pigments minéraux utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

La composition cosmétique selon l'invention peut comprendre également des colorants hydrosolubles ou liposolubles. Les colorants liposolubles sont, par exemple, le rouge Soudan, le DC Red 17, le DC Green 6, le β-carotène, le brun Soudan, le DC Yellow 11, le DC Violet 2, le DC orange 5 et le jaune quinoléine. Les colorants hydrosolubles sont, par exemple, le jus de betterave et le caramel.

Selon un mode particulier, la composition de l'invention comprend au moins une matière colorante particulière choisie parmi les agents de coloration goniochromatiques, les agents de coloration photochromes, les agents fluorescents, les azurants optiques, les laques, les pigments organiques, les pigments composites, et leurs mélanges

### Agents de coloration goniochromatique

Par « agent de coloration goniochromatique », on désigne au sens de la présente invention un agent de coloration permettant d'obtenir, lorsque la composition le contenant est étalée sur un support, un trajet de couleur dans le plan a*b* de l'espace colorimétrique CIE 1976 qui correspond à une variation Dh de l'angle de la teinte h d'au moins 20° lorsque l'on fait varier l'angle d'observation par rapport à la normale entre 0° et 80°, pour un angle d'incidence de la lumière de 45°.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂ Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂ Ag, Al, Au, Cu, Rb, Ti, Ta, W, Zn, MoS₂, cryolithe, alliages, polymères et leurs associations.

Les agents goniochromatiques à structures multicouches sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479 (BASF), DE-A-196 14 637. Ils se présentent sous forme de paillettes, de couleur métallisée.

Les structures multicouches utilisables dans l'invention sont par exemple les structures suivantes : Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/ SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/ mica-oxyde/SiO₂/Fe₂O₃. Selon l'épaisseur des différentes couches, on obtient différentes couleurs. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

Par suite la structure multicouche peut être essentiellement minérale ou organique. Selon l'épaisseur de chacune des différentes couches, on obtient différentes couleurs.

Les pigments goniochromatiques à structure multicouche interférentielle selon l'invention sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479 (BASF), DE-A-196 14 637, et leurs associations. Ils se présentent sous forme de paillettes, de couleur métallisée.

De préférence, le pigment goniochromatique à structure multicouche interférentielle selon l'invention est choisi dans le groupe constitué par les pigments goniochromatiques commerciaux suivants : Infinite Colors de fabriqué ou commercialisé par la société SHISEIDO, Sicopearl Fantastico fabriqué ou commercialisé par la société de BASF, Colorstream fabriqué ou commercialisé par la société de MERCK, Colorglitter fabriqué ou commercialisé par la société de 3M, Chromaflair fabriqué ou commercialisé par la société de FLEX, et Xiraollic et Xirona fabriqués ou commercialisés par la société de MERCK et leurs mélanges.

Comme agent goniochromatique à structure multicouche, on peut citer ceux vendus sous la dénomination "SICOPEARL

Quant aux particules goniochromatiques à cristaux liquides susceptibles d'être mises en oeuvre dans la composition selon l'invention, elles peuvent notamment être à base de polymère susceptible d'être obtenu par polymérisation d'un mélange de monomères comprenant :
- a) au moins un premier monomère A de formule (I) Y1-A1-M1-A2-Y2 dans laquelle
- i) Y1 et Y2 , identiques ou différents, représentent un groupe polymérisable choisi parmi les groupements acrylate ou méthacrylate, un groupement époxy, un groupe isocyanate, hydroxy, vinyléther (-O-CH=CH₂) ou vinylester (-CO-O-CH=CH₂ et
- ii) A1 et A2, identiques ou différents, représentent un groupement de formule -CnH₂n-, dans laquelle n est un nombre entier allant de 0 à 20 et un ou plusieurs groupes méthylène dudit groupement -CnH₂n- pouvant être remplacés par un ou plusieurs atomes d'oxygène, et
- iii) M1 désigne un groupement de formule générale (I') -R₁-X₁-R₂-X₂-R₃-X₃-R₄-, dans laquelle R₁, R₂, R₃ et R₄ , identiques ou différents, désignent un groupement divalent choisi parmi -O-, -COO-, -CONH-, -CO-, -S-, -C≡C-, -CH=CH-, -N=N-, -N=N(O)-, et -R₂-X₂-R₃- ou -R₂-X₂- ou -R₂-X₂-R₃-X₃- pouvant également être une liaison covalente simple, et X₁,X₂,X₃ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B1 et/ou B2 et/ou B3, ledit hétéroarylène contenant de 1 à 3 hétéroatomes choisis parmi les atomes O, N et S, les groupements cycloalkylènes ayant de 3 à 10 atomes de carbones éventuellement substitués par -B1 et/ou -B2 et/ou - B3, -B1, -B2, et -B3 , identiques ou différents, étant choisis parmi les groupes alkyle en C₁-C₂₀, alkoxy en C₁-C₂₀, alkylthio en C₁-C₂₀, alkyl(C₁-C₂₀) carbonyl, alkoxy(C₁-C₂₀) carbonyl, alkyl(C₁-C₂₀) thiocarbonyl, -OH, -F, -Cl, -Br, -I, -CN, -NO₂, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,
et
- b) au moins un deuxième monomère B chiral de formule (II) V1-A'1-W1-Z-W2-A'2-V2, dans laquelle
- i) V1 et V2 , identiques ou différents, désignent un groupement choisi parmi un groupement acrylate ou méthacrylate, un groupement époxy, un groupement vinyléther ou vinylester, un groupement isocyanate, un alkyle en C₁-C₂₀, un alkoxy en C₁-C₂₀, un alkylthio en C₁-C₂₀, un alkoxy(C₁-C₂₀) carbonyl, un alkyl(C₁-C₂₀)thiocarbonyle, -OH, -F, -Cl, -Br, -I, -CN, -NO2, formyle, acétyle et des groupements alkyle, alkoxy ou alkylthio, ayant de 1 à 20 atomes de carbone, et interrompus par un ou plusieurs atome(s) d'oxygène ou un ou plusieurs atome(s) de soufre ou un ou plusieurs groupe(s) ester (-CO-O-),
   et au moins V1 ou V2 désigne un groupe polymérisable choisi parmi les groupements acrylate ou méthacrylate, un groupement époxy, un groupe isocyanate, hydroxy, vinyléther (-O-CH=CH₂) ou vinylester (-CO-O-CH=CH₂,
- ii) A'1 et A'2 , identiques ou différents, représentent un groupement de formule -CₙH₂ₙ-, dans laquelle n est un nombre entier allant de 0 à 20 et un ou plusieurs groupes méthylène dudit groupement CₙH₂ₙ pouvant être remplacés par un ou plusieurs atomes d'oxygène, et
- iii) W1 et W2 désignent un groupement divalent de formule générale R'₁-X'₁-R'₂-X'₂-R'₃- dans laquelle R'₁ , R'₂ et R'₃, identiques ou différents, désignent un groupement divalent choisi parmi -O-, -COO-, -CONH-, -CO-, -S-, -C≡C-, -CH=CH-, -N=N-, -N=N(O)-, et R'₁, R'₂ , R'₃ ou R'₂-X'₂ peut également être une liaison covalente simple, et X'₁ et X'₂ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B'1 et/ou B'2 et/ou B'3, ledit hétéroarylène contenant de 1 à 3 hétéroatomes choisis parmi les atomes O, N et S, les groupements cycloalkylènes ayant de 3 à 10 atomes de carbones éventuellement substitués par -B'1 et/ou -B'2 et/ou -B'3, -B'1, -B'2, et -B'3, identiques ou différents, étant choisis parmi les groupes alkyle en C₁-C₂₀, alkoxy en C₁-C₂₀, alkylthio en C₁-C₂₀, alkyl(C₁-C₂₀)carbonyl, alkoxy (C₁-C₂₀)carbonyl, alkyl(C₁-C₂₀)thiocarbonyl, -OH, -F, -Cl, -Br, -I, -CN, -NO₂, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,
   et Z désigne un groupement chiral divalent comprenant au moins 4 atomes de carbone, notamment de 4 à 20 atomes de carbone, et mieux de 4 à 10 atomes de carbone, (le groupement chiral divalent comprenant au moins un carbone asymétrique, notamment un ou deux carbones asymétriques, et en particulier deux carbones asymétriques) et en particulier un groupe chiral divalent issu du groupe des dianhydrohexites, hexoses, pentoses, les dérivés binaphthyle (groupements binaphtyle), les dérivés biphényle (groupements biphényle), les dérivés d'acides tartriques ou des glycols optiquement actifs.

De préférence, les particules de polymère à cristaux liquides ont une plus grande taille allant de 1 µm à 3 mm, et de préférence allant de 30 µm à 500 µm. Ces particules sont avantageusement en forme de plaquettes.

De tels polymères et leurs particules sont décrits dans la demande EP-A-1046692.

Comme particules de polymère à cristaux liquides, on peut notamment utiliser celles connues sous le nom CTFA Polyacrylate-4 et vendus sous les dénominations « HELICONE^{®} HC Sapphire », « HELICONE^{®} HC Scarabeus », « HELICONE^{®} HC Jade », « HELICONE^{®} HC Maple », « HELICONE^{®} HC XL Sapphire », « HELICONE^{®} HC XL Scarabeus », « HELICONE^{®} HC XL Jade », « HELICONE^{®} HC XL Maple » par la société WACKER.

### Agents de coloration photochromes

Un agent de coloration photochrome est un agent ayant la propriété de changer de couleur lorsqu'il est éclairé par de la lumière ultraviolette et de reprendre sa couleur initiale lorsqu'il n'est plus éclairé par cette lumière ou encore de passer d'un état non coloré à un état coloré et inversement. En particulier, cet agent présente des couleurs différentes selon qu'il est éclairé par de la lumière naturelle ou de la lumière artificielle.

Les agents photochromes utilisables dans l'invention sont en particulier ceux décrits dans les documents JP-A-09/165532, EP-A-709728, JP-A-07/258580, JP-A-07/223816, EP-A-624553, JP-A-08/337422, JP-A-07/025617, EP-A-359909.

Plus précisément, les agents coloration photochromes utilisables dans l'invention sont les spiro-oxazines et leurs dérivés comme les spiroindolino-naphto-oxazines, les spironaphtoxazines, le naphtopyrane et ses dérivés, les spiropyrannes, comme les indolinospirobenzopyrannes, les nitrobenzylpyridines, les spirolanes, les oxydes de titane ou de zinc dopé par du fer. A titre d'exemple, on peut citer les agents photochromes du type dérivés de naphtopyrane vendus par la société PPG sous les références Photosol 5-68 Photochromic Dye, Photosol 7-49 Photochromic Dye, Photosol 7-106 Photochromic Dye, Photosol 0265 Photochromic Dye, Photosol 0272 Photochromic Dye, ces agents présentant deux couleurs différentes selon qu'ils sont excités ou non par des U.V. On peut aussi utiliser des aluminosilicates dopés notamment par les groupements S, Se, SO₄²⁻, WO₄²⁻ ou OH ou encore par des ions métalliques notamment de Fe, Cr, Mn, Co, Ni, tels que ceux décrits dans la demande EP-A-847751.

### Agents (ou substance) fluorescents

Les agents fluorescents sont bien connus de l'homme du métier. Ilss peuvent être des pigments ou des colorants. Par pigments, on entend des particules, minérales ou organiques, insolubles dans la composition. Par colorant, on entend des composés chimiques solubilisés dans la composition. Les colorants peuvent être hydrosolubles ou liposolubles. Les substances fluorescentes sont par exemple décrites dans « Luminescent materials (fluorescent daylight), Encyclopedia of Chemical Technology, Kirk-Othmer", vol 14, p. 546-569, 3ème édition, 1981, Wiley.

Au sens de la présente invention, on entend par agent fluorescent une substance qui, sous l'effet de rayons ultraviolet et/ou de la lumière visible, réémet dans le visible la portion de lumière qu'elle a absorbé sous la même couleur que celle qu'elle reflète naturellement. La couleur réfléchie naturellement est ainsi renforcée par la couleur réémise et apparaît extrêmement brillante.

Comme agent fluorescent, on peut utiliser des substances fluorescentes inorganiques comme celles décrites dans la demande JP05-117127 et en particulier les substances fluorescentes inorganiques à base d'oxyde de zinc.

Comme agents fluorescent , on peut également utiliser des substances fluorescentes organiques comme les pigments fluorescents à la lumière du jour : ces pigments sont généralement fabriqués à partir de colorants fluorescents qui sont préalablement dissous dans une résine support afin d'obtenir une solution solide laquelle est ensuite broyée en une poudre de particules de résine présentant des propriétés fluorescentes. La préparation de tels pigments fluorescents est écrite dans EP 0 370 470, US 2, 851, 424, US 3, 711, 604, US 3, 856, 550 et US 2, 938, 878.

Les pigments fluorescents convenant particulièrement bien à la présente invention peuvent ainsi être choisis parmi les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

Comme pigments fluorescents convenant particulièrement bien à la présente invention, on peut citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

### Azurants optiques

Les agents fluorescents peuvent être aussi choisi parmi les azurants optiques, qui sont des substances fluorescentes organiques blanches.

Les azurants optiques sont des composés bien connus de l'homme du métier. De tels composés sont décrits dans "Fluorescent Whitening Agent, Encyclopedia of Chemical Technology, Kirk-Othmer", vol 11, p. 227-241, 4ème édition, 1994, Wiley.

On peut plus particulièrement les définir comme des composés qui absorbent essentiellement dans l'UVA entre 300 et 390 nm et réémettent essentiellement entre 400 et 525 nm.

Leur effet éclaircissant repose plus particulièrement sur une émission d'énergie comprise entre 400 et 480 nm, ce qui correspond à une émission dans le bleu du domaine visible, qui contribue, quand cette émission a lieu sur la peau, à l'éclaircir visuellement.

Sont notamment connus à titre d'azurants optiques les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène, les dérivés de porphyrine et leurs mélanges.

De tels composés sont largement commercialisés. Il s'agit notamment des dérivés suivants :
- le dérivé stilbénique de naphto-triazole vendu sous la dénomination commerciale « Tinopal GS », le di-styryl-4,4' biphényle sulfonate di-sodique (nom CTFA : disodium distyrylbiphenyl disulfonate) vendu sous la dénomination commerciale « Tinopal CBS-X », le dérivé cationique d'aminocoumarine vendu sous la dénomination commerciale « Tinopal SWN CONC. », le 4,4'-bis[(4,6-dianilino-1,3,5-triazin-2-yl)amino]stilbène-2,2'-disulfonate de sodium vendu sous la dénomination commerciale « Tinopal SOP », l'acide 4,4'-bis-[(4-anilino-6-bis(2-hydroxyéthyl)amino-1,3,5-triazin-2-yl)amino]stilbène-2,2'-disulfonique vendu sous la dénomination commerciale « Tinopal UNPA-GX », le 4,4'-bis-[anilino-6-morpholine-1,3,5-triazin-2-yl)amino]stilbène vendu sous la dénomination commerciale « Tinopal AMS-GX », le 4,4'-bis-[(4-anilino-6-(2-hydroxyéthyl)méthyl amino-1,3,5-triazin-2-yl)amino]stilbène-2,2'- disodium sulfonate vendu sous la dénomination commerciale « Tinopal 5BM-GX », tous par la société CIBA Spécialités Chimiques ;
- le 2,5 thiophène di-yl bis(5 ter-butyl-1,3 benzoxazole) vendu sous la dénomination commerciale « Uvitex OB » par la société CIBA ;
- le dérivé anionique du di-aminostilbène en dispersion dans l'eau vendu sous la dénomination commerciale « Leucophor BSB liquide » par la société CLARIANT ; et
- leurs mélanges.

Les azurants optiques utilisables dans la présente invention peuvent aussi se présenter sous la forme de copolymères, par exemple d'acrylates et/ou de méthacrylates, greffés par des groupements azurants optiques comme décrits dans la demande FR 99 10942.

### Laques organiques

Les laques organiques peuvent être choisies, de façon connue, parmi les sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane. Ces colorants comportent généralement au moins un groupe acide carboxylique ou sulfonique.

Les laques organiques peuvent aussi être supportées par tout support compatible tel qu' un support minéral comme les particules d'alumine, d'argile, de zircone ou d'oxydes métalliques, notamment d'oxyde de zinc ou d'oxyde de titane, de talc, de carbonate de calium, de sulfate de barium. De préférene, le support minéral est choisi parmi l'alumine, l'oxyde de titane et le sulfate de barium.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake, et leurs mélanges.

Les composés chimiques correspondant à chacun des pigments organiques cités précédemment sont mentionnés dans l'ouvrage "International Cosmetic Ingredient Dictionnary and Handbook", Edition 1997, pages 371 à 386 et 524 à 528, publié par "The Cosmetic, Toiletry, and Fragrance Association", dont le contenu est incorporé dans la présente demande à titre de référence.

### Pigments organiques

On peut citer notamment les pigments organiques de colorants azoïques, anthraquinoniques, indigoïdes, xanthéniques, pyréniques, quinoliniques, de triphénylméthane, de fluorane.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6, D&C black 2 (carbon black) et bleu de phtalocyanine, et leurs mélanges.

### Pigments composites

Selon un mode particulièrement préféré, la composition de l'invention contient comme matière colorante particulière associée à l'oxychlorure de bismuth dispersé dans au moins une huile spécifique telle que définie ci-dessus, au moins un pigment composite tel que décrit ci-après.

### Structure

Un pigment composite selon l'invention peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique. Ce liant peut avantageusement agir sans la formation de liaisons covalentes.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite selon l'invention peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

### Noyau inorganique

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau inorganique peut présenter une taille moyenne comprise entre environ 5 nm et environ 100 nm, voire entre environ 5 nm et environ 75 nm, par exemple entre environ 10 nm et environ 50 nm, notamment autour de 20 ou 25 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique).

Le noyau inorganique peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

En particulier le noyau inorganique dudit pigment composite comporte un oxyde métallique choisi parmi un oxyde de titane, de zirconium, de cérium, de zinc, de fer, de bleu ferrique, de chrome et d'aluminium, en particulier un oxyde de titane.

Selon un mode particulier de l'invention, le noyau inorganique est un oxyde de titane.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g.

La surface spécifique est par exemple comprise entre 30 et 70 m²/g, par exemple voisine de 50 m²/g, notamment dans le cas de noyaux de TiO₂ de taille voisine de 20 ou 25 nm.

Le noyau inorganique peut être coloré, le cas échéant.
L'indice de réfraction du noyau inorganique est avantageusement supérieur ou égal à 2, voir supérieur ou égal à 2,1 ou 2,2.

La proportion massique du noyau inorganique au sein du pigment composite peut excéder 50 %, étant par exemple comprise entre 50 et 70 %, ou 50 et 60 %.

### Matière colorante organique

La matière colorante organique peut être choisie par exemple parmi les composés particulaires insolubles dans le milieu physiologiquement acceptable de la composition. La matière colorante organique peut comporter par exemple des pigments organiques qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels organiques insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6, D&C black 2 (carbon black) et bleu de phtalocyanine, et leurs mélanges.

Selon un mode particulier, on utilise le pigment organique D&C Red n° 7.

Selon un autre mode, on utilise le pigment organique D&C Red n° 28.

Selon un autre mode particulier, on utilise le pigment organique FD&C Yellow n° 5.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake, et leurs mélanges.
Selon un mode particulier, on utilise la laque organique FD&C Blue n° 1 Aluminium lake ou D&C Blue n° 1 Aluminium lake.

Selon un autre mode particulier, on utilise la laque organique D&C Red n° 28 Aluminium lake.

Selon un autre mode, on utilise la laque organique FD&C Yellow n° 5 Aluminium lake.

Les composés chimiques correspondant à chacune des matières colorantes organiques citées précédemment sont mentionnés dans l'ouvrage « International Cosmetic Ingrédient Dictionnary and Handbook », Edition 1997, pages 371 à 386 et 524 à 528, publié par « The Cosmetic, Toiletry, and Fragrance Association », dont le contenu est incorporé dans la présente demande par référence.

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

Dans certains exemples de réalisation, la teneur totale en matière organique colorante de la composition est inférieure ou égale à 10 % en masse par rapport au poids total de la composition.
La matière colorante organique représente par exemple entre 30 et 50 % en masse du pigment composite par rapport au poids total de celui-ci, par exemple entre 30 et 40 %.

### Liant

Le liant peut être de tout type dès lors qu'il permet à la matière colorante organique d'adhérer à la surface du noyau inorganique.

Selon un mode particulier et préféré, le liant est organique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

De préférence, le liant organique est choisi parmi les composés siliconés.

Le composé siliconé peut être choisi parmi une liste non limitative comprenant notamment :
- les organosilanes (1) obtenus à partir d'alkoxysilanes,
- les polysiloxanes (2) modifiés ou non choisis parmi une liste non limitative comprenant :
- les polysiloxanes modifiés (2A) comprenant au moins un radical choisi parmi, notamment, les polyéthers, les polyesters et les composés époxy (ils seront appelés « polysiloxanes modifiés »),
- les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés organosilanes (1) peuvent être obtenus à partir de composés alkoxysilanes représentés par la formule (I) :

R¹ₐ Si X₄₋ₐ (I)

dans laquelle :
- R¹ représente C₆H₅-, (CH₃)₂ CH-CH₂- ou un radical de type C_{b} H_{2b+1}- (où b varie de 1 à 18),
- X représente CH₃O- ou C₂H₅O-, et
- a varie de 0 à 3.

Des exemples spécifiques de composés alkoxysilanes peuvent inclure les alkoxysilanes choisis parmi : le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriéthoxysilane.

Les polysiloxanes (2) peuvent notamment répondre à la formule (II) : 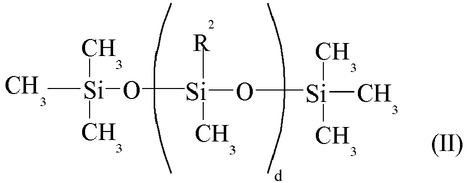 dans laquelle R² représente H- ou CH₃- et d varie de 15 à 450.

Parmi ces polysiloxanes, ceux pour lesquels R² représente H sont préférés.
Les polysiloxanes modifiés (2A) peuvent notamment répondre aux formules suivantes :
- (a¹) polysiloxanes modifiés portant des polyéthers, représentés par la formule (III) 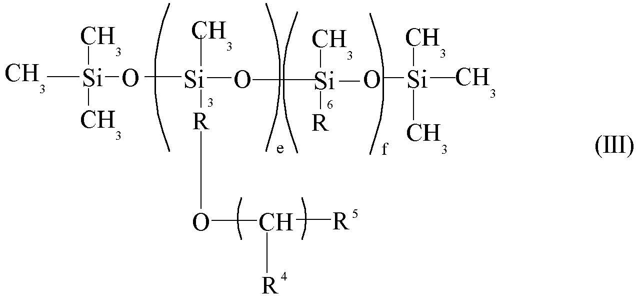 dans laquelle R³ représente -(CH₂)ₕ- ; R⁴ représente -(CH₂)ᵢ- CH₃ ; R⁵ représente -OH, - COOH, -CH = CH₂, -C (CH₃) = CH₂ ou -(CH₂)ⱼ- CH₃ ; R⁶ représente -(CH2)ₖ- CH₃ ; g et h variant indépendamment de 1 à 15 ; j et k variant indépendamment de 0 à 15 ; e variant de 1 à 50 et f variant de 1 à 300,
- (a²) polysiloxanes modifiés portant des polyesters, représentés par la formule (IV) : 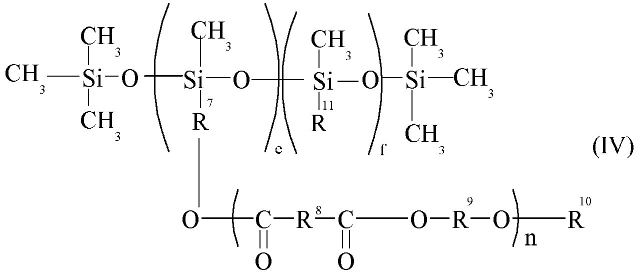 dans laquelle R⁷, R⁸ et R⁹ représentent indépendamment -(CH₂)_{q}- ; R¹⁰ représente -OH ; -COOH, -CH = CH₂, -C(CH₃) = CH₂ ou -(CH₂)ᵣ- CH₃ ; R¹¹ représente -(CH₂)ₛ- CH₃ ; n et q variant indépendamment de 1 à 15, r et s variant indépendamment de 0 à 15 ; e variant de 1 à 50 et f variant de 1 à 300,
- (a³) polysiloxanes modifiés portant des radicaux époxy représentés par la formule (V): 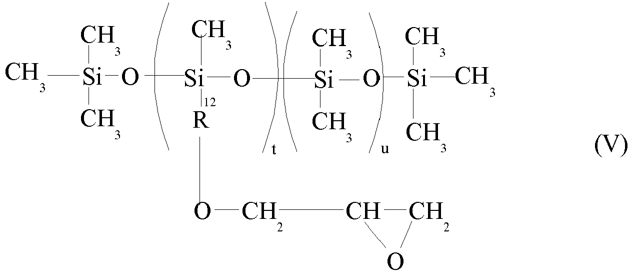
dans laquelle R¹² représente -(CH₂)ᵥ- ; v variant de 1 à 15 ; t variant de 1 à 50 et u variant de 1 à 300 ; ou leurs mélanges.

Parmi les polysiloxanes modifiés (2A), les polysiloxanes modifiés portant des polyéthers de formule (III) sont préférés.

Les polysiloxanes modifiés sur la partie terminale (2B) peuvent répondre à la formule (VI) : 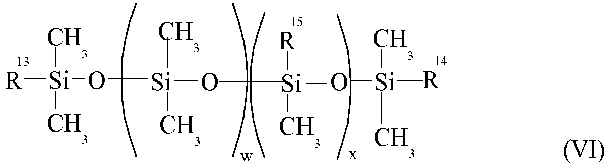 dans laquelle R¹³ et R¹⁴ peuvent représenter -OH, R¹⁶-OH ou R¹⁷-COOH, indépendamment l'un de l'autre ; R¹⁵ représente -CH₃ ou -C₆H₅ ; R¹⁶ et R¹⁷ représentent -(CH₂)y- ; y variant de 1 à 15 ; w variant de 1 à 200 et x variant de 0 à 100.

Parmi ces polysiloxanes modifiés sur au moins une extrémité, ceux portant au moins radical (R¹⁶ et/ou R¹⁷) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

CF₃(CF₂)_{z}CH₂CH₂ (R¹⁸)ₐ SiX₄₋ₐ (VII)

dans laquelle :
- R¹⁸ représente CH₃-, C₂H₅-, CH₃O- ou C₂H₅O-,
- X représente CH₃O- ou C₂H₅O-,
- Z varie de 0 à 15 et a varie de 0 à 3.

Les fluoroalkylsilanes peuvent notamment être choisis dans une liste non limitative comprenant notamment le trifluoropropyltriméthoxysilane, le tridécafluorooctyltriméthoxysilane, l'heptadécafluorodécyltriméthoxysilane, l'heptadécafluorodécylméthyldiméthoxysilane, le trifluoropropyltriéthoxysilane, le tridécafluorooctyltriéthoxysilane, l'heptadécafluorodecyltriéthoxysilane, l'heptadécafluorodécylméthyldiéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyltriméthoxysilane.

Les agents couplants à base de silane peuvent être choisis parmi une liste non limitative comprenant notamment le vinyltriméthoxysilane, le vinyltriéthoxysilane, γ-aminopropyltriéthoxysilane, le γ-flycidoxypropyltriméthoxysilane, le γ-mercaptopropyltriméthoxysilane, le γ-méthacryloxypropyltriméthoxysilane, le N-β(aminoéthyl)-γ-aminopropyltriméthoxysilane, le γ-glycidoxypropylméthyldiméthoxysilane, le γ-chloropropyltriméthoxysilane et similaires.

Les agents couplants à base de titanate peuvent être choisis dans la liste comprenant le titanate d'isopropylstéaroyle, le titanate d'isopropyltris(dioctylpyrophosphate), le titanate d'isopropyltri(N-aminoéthyl-aminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium, le diisopropoxymonoéthylacétoacétate d'aluminium, le triéthylacétoacétate d'aluminium, le triacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.
Pour obtenir une couche recouvrant les noyaux inorganiques uniformément, le liant est de préférence dans un état liquide ou soluble dans l'eau ou dans différents solvants. Selon un mode particulier, le liant organique est un polymethylhydrogenesiloxane.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2.

La proportion relative massique de liant peut être inférieure ou égale à 5 %, voire à 3 %, par rapport au poids total du pigment composite.

### Préparation du pigment composite

Le pigment composite peut être préparé partout procédé approprié, par exemple un procédé mécano-chimique ou un procédé de précipitation en solution, avec dissolution d'une substance colorante organique et précipitation à sa surface du noyau.

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184426.

Dans un exemple de mise en oeuvre, on commence par mélanger les particules destinées à constituer le noyau inorganique avec le liant.

De manière à ce que le liant adhère uniformément à la surface du noyau inorganique, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau inorganique, la matière colorante organique est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux inorganiques avec le liant ou de la matière colorante organique avec les noyaux inorganiques recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement. Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante organique est dissoute dans l'éthanol, les noyaux inorganiques sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

Selon un mode particulier, le pigment composite est choisi parmi des pigments composites comportant :
- un noyau inorganique comportant un oxyde de titane,
- avantageusement un liant siliconé, et
- au moins un enrobage au moins partiel d'au moins une matière colorante organique choisie parmi les laques.

Comme exemples de pigments composites utilisables selon l'invention, seuls ou en mélange, on peut citer notamment :
- dioxyde de titane (CI77891), laque bleue FD&C Blue Aluminium lake (CI42090) et polymethylhydrogensiloxane (58.1/40.7/1.2)
- dioxyde de titane (CI77891), D&C Red n° 7 (CI15850) et polymethylhydrogensiloxane (65.8/32.9/1.3)
- dioxyde de titane (CI77891), D&C Red n° 28 (CI45410) et polymethylhydrogensiloxane (65.8/32.9/1.3)
- dioxyde de titane (CI77891), laque jaune FD&C Yellow 5 Aluminium lake (CI191140) et polymethylhydrogensiloxane (65.8/32.9/1.3),
- dioxyde de titane (CI77891), D&C Red n° 7 et D&C Red n° 28 Aluminium lake (Cl15850 et CI45410) et polymethylhydrogensiloxane.

Selon un mode particulier, le pigment composite est choisi parmi des pigments composites comportant :
- un noyau inorganique comportant un oxyde de titane
- avantageusement un liant siliconé, et
- au moins un enrobage au moins partiel d'au moins une matière colorante organique choisie parmi les laques, en particulier celles de dénomination D&C Blue n° 1 Aluminium lake, FD&C Blue n° 1 Aluminium lake, FD&C Red n° 4 Aluminium lake, FD&C Red n° 40 Aluminium lake, Red n° 28 Aluminium lake, ou les pigments organiques en particulier ceux de dénomination D&C Red n° 7, D&C Red n° 28, et leurs mélanges.

Selon un mode particulier, la composition selon l'invention comprend un pigment composite. A titre d'exemple, on utilise le pigment composite comprenant dioxyde de titane (CI77891), laque bleue FD&C Blue Aluminium lake (CI42090) et polymethylhydrogensiloxane (58.1/40.7/1.2).

Selon un mode préféré, on utilise un premier pigment composite oxyde de titane/ Blue n° 1 Aluminium lake et un second pigment composite oxyde de titane/ Red n° 28 Aluminium lake et D&C Red n° 7.

Cette association particulière de pigments composites permet de conférer un éclat naturel et frais avec un effet coloriel naturel à la peau sur laquelle la composition de l'invention est appliquée, outre l'effet de lumière apporté par la pré-dispersion d'oxychlorure de bismuth décrite précédemment.

La ou les matières colorantes particulières précédemment décrites, choisie(s) parmi les agents de coloration goniochromatiques, les agents de coloration photochromes, les agents fluorescents, les azurants optiques, les laques, les pigments organiques, les pigments composites, et leurs mélanges, est (sont) présente(s) dans ladite composition en une teneur allant de 0.001% à 3% , de préférence de 0.05% à 2% en poids par rapport au poids total de ladite composition.

De façon avantageuse, pour une teneur en matière première de mélange d'oxychlorure de bismuth et d'une huile telle que définie précédemment et en particulier de l'hydroxystéarate d'éthyl (2) hexyle, allant de 0,01 à 8% en poids de matière première, par rapport au poids total de ladite composition, on utilisera une teneur en matière colorante particulière, et en particulier en pigment(s) composite(s), allant de 0.001% à 2% , préférentiellement de 0.01% à 1% en poids par rapport au poids total de ladite composition.

Selon un autre mode, pour une teneur en matière première de mélange d'oxychlorure de bismuth et d'une huile telle que définie précédemment et en particulier de l'hydroxystéarate d'éthyl (2) hexyle allant de 8 à 15% en poids de matière première, par rapport au poids total de ladite composition, on utilisera une teneur en matière colorante particulière, et en particulier en pigment(s) composite(s) allant de 0,1 à 3%, préférentiellemnt de 0,5 à 2,5% en poids par rapport au poids total de ladite composition.

En particulier, le rapport pondéral (A)/(B) entre (A) le mélange d'oxychlorure de bismuth et d'huile telle que définie précédemment et (B) le matériau à effet optique, sera supérieur ou égal à 1, en particulier ira de 2 à 15000, notamment de 3 à 500, en particulier de 5 à 100, voire de 10 à 50, et mieux de 15 à 30.

De façon avantageuse, la composition pourra comprendre en outre au moins une matière colorante et/ou réfléchissante additionnelle, distincte de la matière colorante particulière décrite précédemment et de l'oxuchlorure de bismuth dispersé dans une huile spécifique.

Par 'matières réfléchissantes' on désigne au sens de la présente invention des particules dont la taille, la structure, notamment l'épaisseur de la ou des couches qui la constituent et leur natures physique et chimique, et l'état de surface, leur permettent de réfléchir la lumière incidente avec une intensité suffisante pour pouvoir créer à la surface de la composition revendiquée, lorsque cette dernière est appliquée sur le support à maquiller, des points de brillance visibles à l'oeil nu, c'est-à-dire des points plus lumineux qui contrastent avec leur environnement en semblant briller.

Les matières réfléchissantes peuvent troubler la perception visuelle de la courbure du support maquillé, en tendant à empêcher une focalisation visuelle durable, les points de brillance étant susceptibles d'apparaître ou de disparaître de manière aléatoire lorsque le support maquillé et l'observateur sont animés.

Ladite matière colorante additionnelle peut être choisie parmi des matières colorantes organiques ou inorganiques, notamment, de type pigments inorganiques ou nacres classiquement utilisés dans les compositions cosmétiques, des particules à reflets métalliques, des colorants liposolubles ou hydrosolubles, et leurs mélanges.
La matière réfléchissante additionnelle peut être choisie parmi des particules à reflet métallique.

Bien entendu, l'homme du métier ajustera le choix des matières colorantes et/ou réfléchissantes additionnelles et leurs teneurs respectives en fonction de l'effet recherché sans altérer l'effet lumineux et l'éclat apporté par l'association sus-décrite.

Ces matières colorantes et/ou réfléchissantes additionnelles, lorsqu'elles sont présentes, sont en une teneur allant de 0.01 à 30% en poids, de préférence de 0.1 à 20% en poids, notamment de 1 à 10% en poids par rapport au poids total de ladite composition.

Par pigments, il faut comprendre des particules blanches ou colorées, minérales ou organiques, insolubles dans une solution aqueuse, destinées à colorer et/ou opacifier le film résultant.

Comme pigments minéraux utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30. On peut également citer les structures de pigments de type BaSO4/TiO2/FeSO3 sous la référence SILSEEM de Nihon Koken et silice-oxyde de fer XIRONA Le Rouge de Merck.

La matière colorante peut encore comporter un pigment ayant une structure qui peut être, par exemple, de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société BASF, les nacres TIMIRON commercialisées par la société MERCK, les nacres sur base de mica PRESTIGE commercialisées par la société ECKART ; les nacres sur base mica naturel SUNPEARL de la société Sun Chemical, KTZ de la société Kobo et SUNPRIZMA de la société Sun Chemical ; les nacres sur base de mica synthétique SUNSHINE et SUNPRIZMA commercialisées par la société SUN CHEMICAL, et les nacres sur base mica synthétique TIMIRON SYNWHITE commercialisées par la société MERCK.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut, notamment, citer les nacres de couleur or, notamment, commercialisées par la société BASF, sous le nom de Brillant gold 212G (Timica), Gold 222C (Cloisonne), Sparkle gold (Timica), Gold 4504 (Chromalite) et Monarch gold 233X (Cloisonne) ; les nacres bronzes, notamment, commercialisées par la société MERCK sous la dénomination Bronze fine (17384) (Colorona) et Bronze (17353) (Colorona) et par la société BASF sous la dénomination Super bronze (Cloisonne) ; les nacres oranges, notamment, commercialisées par la société BASF sous la dénomination Orange 363C (Cloisonne) et Orange MCR 101 (Cosmica) et par la société MERCK sous la dénomination Passion orange (Colorona) et Matte orange (17449) (Microna); les nacres de teinte brune, notamment, commercialisées par la société BASF sous la dénomination Nu-antique copper 340XB (Cloisonne) et Brown CL4509 (Chromalite) ; les nacres à reflet cuivre, notamment, commercialisées par la société BASF sous la dénomination Copper 340A (Timica) ; les nacres à reflet rouge, notamment, commercialisées par la société MERCK sous la dénomination Sienna fine (17386) (Colorona) ; les nacres à reflet jaune, notamment, commercialisées par la société BASF sous la dénomination Yellow (4502) (Chromalite) ; les nacres de teinte rouge à reflet or, notamment, commercialisées par la société BASF sous la dénomination Sunstone G012 (Gemtone) ; les nacres roses, notamment, commercialisées par la société BASF sous la dénomination Tan opale G005 (Gemtone) ; les nacres noires à reflet or, notamment, commercialisées par la société BASF sous la dénomination Nu antique bronze 240 AB (Timica), les nacres bleues, notamment, commercialisées par la société MERCK sous la dénomination Matte blue (17433) (Microna), les nacres blanches à reflet argenté, notamment, commercialisées par la société MERCK sous la dénomination Xirona Silver et les nacres orangées rosées vert doré, notamment, commercialisées par la société MERCK sous la dénomination Indian summer (Xirona) et leurs mélanges.

Parmi les particules à reflet métallique, on peut citer notamment :
- les particules d'au moins un métal et/ou d'au moins un dérivé métallique,
- les particules comportant un substrat organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple, les bronzes et les laitons) sont des métaux préférés.

Par « dérivés métalliques », on désigne des composés dérivés de métaux, notamment, des oxydes, des fluorures, des chlorures et des sulfures

A titre illustratif de ces particules, on peut citer des particules d'aluminium, telles que celles commercialisées sous les dénominations STARBRITE 1200 EAC^{®} par la société SIBERLINE et METALURE^{®} par la société ECKART.

On peut également citer les poudres métalliques de cuivre ou des mélanges d'alliages, telles les références 2844 commercialisées par la société RADIUM BRONZE, les pigments métalliques, comme l'aluminium ou le bronze, tels que ceux commercialisés sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique, comme des poudres de bronze (alliage cuivre et zinc) enrobé de silice commercialisées sous la dénomination de Visionaire Bright Natural Gold de la société Eckart.

Il peut encore s'agir de particules comportant un substrat de verre comme celles commercialisées par la société NIPPON SHEET GLASS sous les dénominations MICROGLASS METASHINE, XIRONA de la société MERCK, RONASTAR de la société MERCK, REFLECKS de la société BASF, MIRAGE de la société BASF.

L'agent de coloration goniochromatique peut être choisi, par exemple, parmi les structures multicouches interférentielles et les agents de coloration à cristaux liquides.

Des exemples de structures multicouche interférentielles symétriques utilisables dans des compositions réalisées conformément à l'invention sont, par exemple, les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/AlO₃/TiO₂ ; SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER. On peut également citer des paillettes issues de structure multicouche polymérique DISCO de la société GLITTEREX, MICROGLITTER de la société VENTURE CHEMICAL.

### CHARGES

Une composition conforme à l'invention peut également comprendre au moins une charge, de nature organique ou minérale.

Par « charge », il faut comprendre les particules incolores ou blanches, solides de toutes formes, qui se présentent sous une forme insoluble et dispersée dans le milieu de la composition. De nature minérale ou organique, elles permettent de conférer à la composition de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

Parmi les charges minérales utilisables dans les compositions selon l'invention, on peut citer le talc, le mica, la silice, le siloxysilicate de triméthyle, le kaolin, la bentone, le carbonate de calcium et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; la Sunsphere H-33, la Sunsphere H-51 commercialisés par Asahi Glass ; le Chemicelen commercialisé par Asahi Chemical ; les composites de silice et de dioxyde de titane, comme la série TSG commercialisée par Nippon Sheet Glass, et leurs mélanges.

Une charge convenant à l'invention peut être préférentiellement le carbonate de calcium.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon^{®}), la lauroy-lysine, l'amidon, les poudres de polymères de tétrafluoroéthylène, les microsphères creuses de polymères, telles l'EXPANCEL (NOBEL INDUSTRIE), les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore^{®} L 200 (Chemdal Corporation), les microbilles de résine de silicone (Tospearl^{®} de Toshiba, par exemple), les poudres de polyuréthane, en particulier, les poudres de polyuréthane réticulé comprenant un copolymère, ledit copolymère comprenant du triméthylol hexyllactone, comme le polymère d'hexaméthylène di-isocyanate/triméthylol hexyllactone, commercialisé sous la dénomination de PLASTIC POWDER D-400^{®} ou PLASTIC POWDER D-800^{®} par la société TOSHIKI, les microcires de Carnauba, telles que celle commercialisée sous la dénomination de MicroCare 350^{®} par la société MICRO POWDERS, les microcires de cire synthétique, telles que celle commercialisée sous la dénomination de MicroEase 114S^{®} par la société MICRO POWDERS, les microcires constituées d'un mélange de cire de Carnauba et de cire de polyéthylène, telles que celles commercialisées sous les dénominations de Micro Care 300^{®} et 310^{®} par la société MICRO POWDERS, les microcires constituées d'un mélange de cire de Carnauba et de cire synthétique, telles que celle commercialisée sous la dénomination Micro Care 325^{®} par la société MICRO POWDERS, les microcires de polyéthylène, telles que celles commercialisées sous les dénominations de Micropoly 200^{®}, 220^{®}, 220L^{®} et 250S^{®} par la société MICRO POWDERS ; et leurs mélanges.

Selon un mode particulier, la composition comprend en outre au moins un agent à effet de flou. De préférence dans ce cas, il s'agira d'une composition anhydre.

### AGENT A EFFET SOFT FOCUS (EFFET DE FLOU)

Par 'agent à effet soft focus' ou 'agent à effet de flou' selon l'invention, on entend un agent destiné à donner plus de transparence au teint et un effet de flou. En particulier, l'agent à effet soft focus permet à la composition qui le contient d'atténuer, par effet optique, le microrelief cutané, et en particulier les défauts cutanés tels que les taches, les rides, les ridules.

Cet agent peut être choisi notamment parmi les charges minérales, les charges organiques, les agents de colorations composites, les élastomères de silicone, et leurs mélanges.

Les agents à effet de flou susceptibles d'être utilisées dans la composition selon l'invention peuvent notamment comporter ou être constituées de particules présentant une taille moyenne en nombre inférieure ou égale à 15 µm, notamment inférieure ou égale à 10 µm, en particulier inférieure ou égale à 7,5 µm, voire inférieure ou égale à 5 µm, par exemple comprise entre 1 µm et 5 µm.

Par « taille moyenne en nombre », on désigne la dimension donnée par la distribution granulométrique statistique à la moitié de la population, dite D50.
Ces particules peuvent être de toutes formes et en particulier être sphériques ou non sphériques.

Les agents à effet de flou selon l'invention peuvent être de toute nature chimique dans la mesure où ils sont compatibles avec une utilisation en cosmétique et où elles n'affectent pas les propriétés attendues de composition.

Ils peuvent ainsi être choisis parmi les poudres de silice et silicates, notamment d'alumine, les poudres de type polyméthyl méthacrylate (PMMA), le talc, les composites silice/TiO₂ ou silice/oxyde de zinc, les poudres de polyéthylène, les poudres d'amidon, les poudres de polyamides, les poudres de copolymères styrène/acrylique, les élastomères de silicone, et leurs mélanges.

En particulier, l'agent à effet de flou est choisi parmi les poudres de polytétrafluoroéthylène, les poudres de polyuréthane, les microcires de Carnauba, les microcires de cire synthétique, les poudres de résine de silicone , les particules hémisphériques creuses de silicone , les poudres de copolymères acryliques, les microsphères de vinylidène/acrylonitrile/méthacrylate de méthylène expansées , les poudres de polyéthylène, notamment comprenant au moins un copolymère éthylène/acide acrylique, les poudres d'organopolysiloxane élastomérique réticulé, les poudres d'organopolysiloxane élastomérique réticulé enrobées de résine de silicone, les poudres de polyamide, les poudres de silice et silicates, notamment d'alumine, le talc de taille moyenne en nombre inférieure ou égale à 3 microns, les composites silice/TiO₂ , les particules de sulfate de baryum, les particules de nitrure de bore, les particules de silice traitées en surface par une cire minérale 1 à 2 % , les microsphères de silice amorphe, les micro-billes de silice , les poudres composites de talc/TiO₂/alumine/silice, les pigments composites de séricite/TiO2/oxyde de fer brun/silice, les élastomères de silicone, et leurs mélanges.

De préférence, l'agent à effet de flou est choisi parmi un pigment composite de séricite/TiO₂/oxyde de fer brun/silice, une poudre de polyuréthane, un élastomère de silicone, et leurs mélanges.

Des exemples de charges, agents de coloration composites et élastomères de silicone à effet de flou (effet soft focus) sont donnés ci-après.

### Charges à effet soft focus

Selon un mode de réalisation, la composition selon l'invention peut comprendre comme agent à effet de flou, une charge.

En particulier, l'agent à effet de flou est une charge choisie parmi les poudres de polytétrafluoroéthylène, les poudres de polyuréthane, les microcires de Carnauba, les microcires de cire synthétique, les poudres de résine de silicone , les particules hémisphériques creuses de silicone , les poudres de copolymères acryliques, les microsphères de vinylidène/acrylonitrile/méthacrylate de méthylène expansées, les poudres de polyéthylène, notamment comprenant au moins un copolymère éthylène/acide acrylique, les poudres d'organopolysiloxane élastomérique réticulé, les poudres d'organopolysiloxane élastomérique réticulé enrobées de résine de silicone, les poudres de polyamide, les poudres de silice et silicates, notamment d'alumine, le talc de taille moyenne en nombre inférieure ou égale à 3 microns, les composites silice/TiO₂, les particules de sulfate de baryum, les particules de nitrure de bore, les particules de silice traitées en surface par une cire minérale 1 à 2 %, les microsphères de silice amorphe, les micro-billes de silice , les poudres composites de talc/TiO₂/alumine/silice comme par exemple celles vendues sous la dénomination COVERLEAF AR-80^{®} et leurs mélanges.

Comme charges à effet soft focus utilisables selon l'invention, on peut citer notamment :
a) les charges organiques telles que
   - les poudres de polytétrafluoroéthylène, comme par exemple les PTFE CRIDUST 9205F^{®} de CLARIANT de taille moyenne de 8 µm ;
   - les poudres de polyuréthane, telles que les poudres de polyuréthane réticulé comprenant un copolymère, ledit copolymère comprenant du triméthylol hexyllactone, comme le polymère d'hexaméthylène di-isocyanate/triméthylol hexyllactone, commercialisé sous la dénomination de PLASTIC POWDER D-400^{®} ou PLASTIC POWDER D-800^{®} par la société TOSHIKI,
   - les microcires de Carnauba, telles que celle commercialisée sous la dénomination de MicroCare 350^{®} par la société MICRO POWDERS, les microcires de cire synthétique, telles que celle commercialisée sous la dénomination de MicroEase 114S^{®} par la société MICRO POWDERS
   - les poudres de résine de silicone comme par exemple les SILICON RESIN TOSPEARL 145A^{®} de GE SILICONE de taille moyenne de 4,5 µm ;
   - les particules hémisphériques creuses de silicone comme par exemple les NLK 500^{®}, NLK 506^{®} et NLK 510^{®} de TAKEMOTO OIL AND FAT ;
   - les poudres de copolymères acryliques, notamment de poly(méth)acrylate de méthyle, comme par exemple les particules PMMA JURIMER MBI^{®} de NIHON JUNYOKI de taille moyenne de 8 µm, les sphères creuses de PMMA vendues sous la dénomination COVABEAD LH85^{®} par la société WACKHERR, les particules PMMA GANZPEARL GMP0820^{®} de la société GANZ CHEMICAL, et les microsphères de vinylidène/acrylonitrile/méthacrylate de méthylène expansées vendues sous la dénomination EXPANCEL^{®} ;
   - les poudres de polyéthylène, notamment comprenant au moins un copolymère éthylène/acide acrylique, et en particulier constituées de copolymères éthylène/acide acrylique comme par exemple les particules FLOBEADS EA 209^{®} de SUMITOMO de taille moyenne de 10 µm ;
   - les poudres d'organopolysiloxane élastomérique réticulé telles que Dow Corning 9701 Cosmetic Powder^{®} de la société Dow Corning (nom INCI 'dimethicone/vinyl dimethicone crosspolymer') ;
   - les poudres d'organopolysiloxane élastomérique réticulé enrobées de résine de silicone, notamment de résine silsesquioxane, comme décrit par exemple dans le brevet US 5 538 793. De telles poudres d'élastomère sont vendues sous les dénominations KSP-100^{®}, KSP-101^{®}, KSP-102^{®}, KSP-103^{®}, KSP-104^{®} et KSP-105^{®} par la société SHIN ETSU ;
   - les poudres de polyamide, telles que la poudre de Nylon^{®} 12, notamment celle vendue sous le dénomination Orgasol 2002 Extra D Nat Cos^{®} par la société Atochem ;
b) les charges minérales telles que :
   - les poudres de silice et silicates, notamment d'alumine,
   - le talc, en particulier le talc de taille moyenne en nombre inférieure ou égale à 3 microns, par exemple du talc de taille moyenne en nombre de 1,8 micron et notamment celui vendu sous la dénomination commerciale Talc P3^{®} par la société Nippon Talc
   - les composites silice/TiO₂ tels que les composites NPT30K3TA de Nippon SHeet Glass ou STMCAS-152010 de Catalysts & Chemicals, ou silice/oxyde de zinc,
   - les particules de sulfate de baryum,
   - les particules de nitrure de bore,
   - les particules de silice traitées en surface par une cire minérale 1 à 2 % (nom INCI : hydrated silica (and) paraffin) telles que celles commercialisées par la société Degussa,
   - les microsphères de silice amorphe, telles que celles vendues sous la dénomination Sunsphère par exemple de référence H-53^{®} par la société Asahi Glass,
   - les micro-billes de silice telles que celles vendues sous la dénomination SB-700^{®} ou SB-150^{®} par la société Miyoshi,
   - les poudres composites de talc/TiO₂/alumine/silice comme par exemple celles vendues sous la dénomination COVERLEAF AR-80^{®} par la société CATALYST & CHEMICALS ;
      et leurs mélanges.

Selon un mode préféré, la composition comprend au moins une charge organique à effet soft focus choisie parmi les poudres de polytétrafluoroéthylène, les poudres de polyuréthane, les poudres de résine de silicone, les particules hémisphériques creuses de silicone, les poudres de copolymères acryliques, les poudres de polyéthylène, les poudres d'organopolysiloxane élastomérique réticulé , les poudres d'organopolysiloxane élastomérique réticulé enrobées de résine de silicone, notamment de résine silsesquioxane, les poudres de polyamide, et leurs mélanges.

### Agent de coloration à effet soft focus

Selon un autre mode de réalisation, la composition selon l'invention peut comprendre comme agent à effet de flou, un agent de coloration à effet soft focus, de type composite. Cet agent de coloration peut comporter un pigment ayant une structure qui peut être par exemple de type séricite/oxyde de fer brun/dioxyde de titane/silice. Un tel pigment est commercialisé par exemple sous les références COVERLEAF NS ou JS ou MF par la société CHEMICALS AND CATALYSTS.

L'agent de coloration peut encore comporter un pigment ayant une structure qui peut être par exemple de type microsphères de silice contenant de l'oxyde de fer, tel que celui commercialisé par la société MIYOSHI sous la référence PC BALL PC-LL-100 P, ce pigment étant constitué de microsphères de silice contenant de l'oxyde de fer jaune.

Selon un mode préféré, on utilisera un agent de coloration composite ayant une structure de type séricite/oxyde de fer brun/dioxyde de titane/silice tel que celui commercialisé sous la référence COVERLEAF MF par la société CHEMICALS AND CATALYSTS.

### Elastomères de silicone à effet soft focus

Selon un autre mode de réalisation, la composition selon l'invention peut comprendre, comme agent à effet soft focus, au moins un élastomère de silicone ou organopolysiloxane élastomère, de préférence au moins partiellement réticulé.

Les organopolysiloxanes élastomères utilisés dans la composition selon l'invention sont de préférence partiellement ou totalement réticulés. Ils se présentent sous forme de particules. En particulier, les particules d'organopolysiloxane élastomère ont une taille allant de 0,1 à 500 µm, de préférence de 3 à 200 µm et mieux de 3 à 50 µm. Ces particules peuvent avoir toute forme et par exemple être sphériques, plates ou amorphes.

L'organopolysiloxane réticulé élastomère peut être obtenu par réaction d'addition réticulation d'un diorganopolysiloxane contenant au moins un atome d'hydrogène lié à un atome de silicium et d'un diorganopolysiloxane ayant au moins deux groupements à insaturation éthylénique liés à des atomes de silicium distincts, notamment en présence de catalyseur platine ; ou par réaction de condensation réticulation déhydrogénation entre un diorganopolysiloxane à terminaisons hydroxyle et un diorganopolysiloxane contenant au moins un atome d'hydrogène lié à un atome de silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

L'élastomère obtenu peut être un élastomère non-émulsionnant ou un élastomère émulsionnant.

Le terme "non émulsionnant" défini des élastomères organopolysiloxane ne contenant pas de chaîne hydrophile. Le terme "émulsionnant" signifie des élastomères organopoysiloxanes réticulés ayant au moins une chaîne hydrophile.

Les particules d'organopolysiloxane réticulé élastomère peuvent être véhiculées sous forme de gel constitué d'un organopolysiloxane élastomérique inclus dans au moins une huile hydrocarbonée et/ou une huile siliconée. Dans ces gels, les particules d'organopolysiloxane sont souvent des particules non-sphériques.

Les particules d'organopolysiloxane réticulé élastomère peuvent également se présenter sous forme de poudre, notamment sous forme de poudre sphérique, en partiuclier enrobée de résine de silicone, notamment de résine silsesquioxane, comme décrit par exemple dans le brevet US5538793. De tels élastomère sont vendus sous les dénomination "KSP-100", "KSP-101", "KSP-102", "KSP-103", KSP-104", "KSP-105" par la société Shin Etsu.

D'autres organopolysiloxanes réticulés élastomères sous forme de poudres peuvent être des poudres de silicone hybride fonctionnalisée par des groupes fluoroalkyle, notamment vendues sous la dénomination "KSP-200" par la société Shin Etsu ; ou des poudres de silicones hybrides fonctionnalisées par des groupes phényl, notamment vendues sous la dénomination "KSP-300" par la société Shin Etsu.

Selon un mode particulier de l'invention, l'agent à effet soft focus est un élastomère de silicone émulsionnant, c'est-à-dire un organopolysiloxane élastomérique (élastomère de silicone) partiellement ou totalement réticulé, comprenant au moins une chaîne hydrophile.

L'élastomère de silicone émulsionnant peut être choisi parmi les élastomères de silicone polyoxyalkylénés.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US5236986 et US5412004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxanelaurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.
L'élastomère de silicone polyoxyalkyléné selon l'invention est véhiculé sous forme de gel dans au moins une huile hydrocarbonée et/ou une huile siliconée. Dans ces gels, l'élastomère polyoxyalkyléné est sous forme de particules non-sphériques.

Des élastomères polyoxyalkylénés sont notamment décrits dans les brevets US5236986, US5412004, US5837793, US5811487 dont le contenu est incorporé par référence.
Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société Shin Etsu, "DC9010", "DC9011" par la société Dow Corning.
Selon un mode préféré, on utilise l'élastomère de silicone polyoxyalkyléné vendu sous la référence KSG-210 par la société Shin Etsu.

L'élastomère de silicone émulsionnant peut être également choisi parmi les élastomères de silicone polyglycérolés.

L'élastomère de silicone polyglycérolé est un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxanelaurylméthylsiloxane à terminaisons triméthylsiloxy.

Le composé (B) peut être un composé polyglycérolé répondant à la formule (B') suivante

CₘH₂ₘ₋₁ -O-[Gly]n-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé selon l'invention est généralement mélangé avec au moins une huile hydrocarbonée et/ou une huile siliconée pour former un gel. Dans ces gels, l'élastomère polyglycérolé est souvent sous forme de particules non-sphériques.

De tels élastomères sont notamment décrits dans la demande de brevet WO2004/024798.

Comme élastomères de silicone polyglycérolés, on peut utiliser ceux vendus sous les dénominations "KSG-710", "KSG-810", "KSG-820", "KSG-830", "KSG-840" par la société Shin Etsu.

Selon un mode particulier de réalisation, la composition selon l'invention comprend au moins un élastomère de silicone non sphérique non émulsionnant mélangé avec au moins une huile hydrocarbonée et/ou une huile siliconée pour former un gel.

Selon un mode préféré, on utilise l'élastomère de silicone polyglycérolé vendu sous la référence KSG-710 par la société Shin Etsu.

Selon un autre mode de réalisation de l'invention, l'agent à effet soft focus utilisé est un élastomère de silicone non émulsionnant.

Le terme élastomères de silicone "non émulsionnant" définit des élastomères organopolysiloxane ne contenant pas de chaîne hydrophile telle que motifs polyoxyalkylènes ou polyglycérolés.

L'élastomère de silicone non émulsionnant est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique organopolysiloxane, notamment en présence de catalyseur organopéroxyde ; ou par réticulation 'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.

Les organopolysiloxanes (A2) peuvent être choisi parmi les méthylvinylsiloxanes, les copolymères méthylvinylsiloxane-diméthylsiloxanes, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).

Avantageusement, la somme du nombre de groupements éthyléniques par molécule du composé (A2) et le nombre d'atomes d'hydrogène liés au silicum par molécule du composé (B2) est d'au moins 4.

Le composé (B2) peut être sous toute structure moléculaire, notamment de structure chaîne linéaire, chaîne ramifiée, structure cyclique.

Le composé (B2) peut avoir une viscosité à 25 °C allant de 1 à 50 000 centistokes, notamment pour être bien miscible avec le composé (A).

Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.

Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

Selon un mode préféré de réalisation, l'élastomère de silicone non émulsionnant est mélangé avec au moins une huile hydrocarbonée et/ou une huile siliconée pour former un gel. Dans ces gels, l'élastomère non émulsionnant est sous forme de particules non-sphériques.

Comme élastomères non émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44", "USG-105, USG-106 par la société Shin Etsu, "DC 9040", "DC9041", "DC 9509", "DC9505", "DC 9506", DC5930, DC9350, DC9045, DC9043 par la société Dow Corning, "GRANSIL" par la société Grant Industries, "SFE 839" par la société General Electric.

Selon un mode préféré, on utilisera comme élastomère non émulsionnant celui vendu sous la référence DC9045 par la société Dow Corning.

Avantageusement, la composition selon l'invention peut contenir au moins deux agents à effet soft focus choisis parmi les poudres de polyuréthane, les élastomères de silicones, les agents de coloration composites et leurs mélanges.

En particulier, la composition selon l'invention peut contenir un élastomère de silicone émulsionnant et un élastomère de silicone non émulsionnant.

Selon la nature de l'agent à effet de flou, l'homme du métier ajustera sa teneur dans la composition, pour obtenir l'effet recherché.

A titre d'exemple, la charge à effet de flou peut être présente dans la composition selon l'invention en une teneur allant de 0,1 à 80 % en poids et notamment allant de 1 % à 70 % en poids par rapport au poids total de la composition, notamment entre 5 et 30 %, par exemple de l'ordre de 8 %.

L'agent de coloration à effet de flou peut être présent dans la composition selon l'invention en une teneur allant de 0,1 à 30 % en poids et notamment allant de 1 % à 20 % en poids par rapport au poids total de la composition, notamment entre 5 et 15 %, par exemple de l'ordre de 8 %.

L'élastomère de silicone à effet de flou peut être présent dans la composition selon l'invention en une teneur en matière active allant de 0,01 % à 8 % en poids, par rapport au poids total de la composition, de préférence allant de 2 % à 7 % en poids, et plus préférentiellement allant de 3 % à 6 % en poids.

### ADDITIFS

Une composition cosmétique selon l'invention peut également comprendre en outre tout additif usuellement utilisé dans le domaine concerné, par exemple choisi parmi des agents filmogènes, et le cas échéant, des auxiliaires de filmification, des gommes, des polymères semi-cristallins, des agent antioxydants, des vitamines, des huiles essentielles, des conservateurs, des parfums, des neutralisants, des agents antiseptiques, des agents protecteurs contre les UV, et leurs mélanges.

Une composition selon l'invention peut, notamment, se présenter sous la forme d'un produit de soin et/ou de maquillage de la peau, en particulier de la peau du corps ou du visage.

Selon un mode de réalisation, une composition de l'invention peut avantageusement se présenter sous la forme d'un ond de teint ou d'un correcteur de teint.
Une composition de l'invention peut être obtenue par tout procédé de préparation connu de l'homme de l'art.

La présente invention sera mieux comprise au moyen des exemples qui suivent.

Ceux-ci ne sont présentés qu'à titre d'illustration de l'invention et ne doivent pas être interprétés comme limitant la portée de celle-ci.

Les valeurs sont exprimés en % massiques.

### EXEMPLES

### Exemple 1 : Crème (émulsion E/H)

| | 1A (comparatif) | 1B (invention) | 1C (invention) |
|---|---|---|---|
| Eau | 36,9 | 35,335 | 33,6 |
| Cyclopenta dimethylsiloxane (DOW | 27,4 | 25,9 | 24,57 |
| CORNING 245 FLUID de Dow Corning) | | | |
| mélange polyglycéryl-4 isostéarate/cétyl diméthicone copolyol/hexyllaurate vendu sous la dénomination ABIL WE 09 par la société Goldschmidt | 9 | 8,55 | 8,1 |
| Ethanol | 5 | 4,75 | 4,5 |
| Glycérine | 5 | 4,75 | 4,5 |
| Isododécane | 5 | 4,75 | 4,5 |
| Mélange de Poly Diphenyl Dimethylsiloxane (Gomme - Visco> 1000000 CST - PM: 600000) et de Cyclopenta Dimethylsiloxane (15/85) vendu sous la dénomination SILBIONE par BLUESTAR | 4 | 3,8 | 3,6 |
| Phenyl Trimethicone | 1,6 | 1,37 | 1,14 |
| Hectorite modifiée Distearyl Dimethyl Ammonium (Bentone 38 VCG de Elementis) | 1,2 | 1,14 | 1,08 |
| Amidon de Mais Esterifié par Anhydride Octenylsuccinique, Sel d'Aluminium (Dry Flo Plus de Akzo Nobel) | 1 | 0,95 | 0,9 |
| 4-Methoxycinnamate de 2-Ethyl Hexyle protégé (Parsol MCX de DSM Nutritional Products) | 1 | 0,95 | 0,9 |
| Sulfate de magnésium | 0,7 | 0,665 | 0,63 |
| Conservateurs | 0,7 | 0,665 | 0,63 |
| Gomme de cellulose | 0,5 | 0,475 | 0,45 |
| Tristearine et Acetylated Glycol Stearate | 0,5 | 0,475 | 0,45 |
| Micro-Sphères Chlorure de Vinylidene/Acrylonitrile/PMMA A L'Iso-Butane expansées (Expancel 551 de Expancel) | 0,3 | 0,285 | 0,27 |
| Nylon-12 | 0,2 | 0,19 | 0,18 |
| Dispersion d'oxychlorure de bismuth dans hydroxystéarate d'éthyl (2) hexyle (70 :30) (Timiron Liquid Silver^{®}) de Merck | 0 | 5 | 10 |

### Mode opératoire :

On introduit la dispersion d'oxychlorure de bismuth dans hydroxystéarate d'éthyl (2) hexyle dans la phase grasse. On prépare la phase aqueuse, puis, en utilisant une défloculeuse sur Rayneri, on procède à l'émulsion, sous forte agitation et à température ambiante.

### Evaluation maquillage:

Les compositions sont appliquées sur plusieurs panels de 10 femmes de 25 à 55 ans. Les évaluations se font par maquillage du visage desdites femmes (auto-évaluations, évaluations par les formulateurs, évaluation par des esthéticiennes). Les formules sont donc comparées entre elles sur les critères de maquillage et notamment la luminosité.

Les formules contenant la dispersion d'oxychlorure de bismuth dans hydroxystéarate d'éthyl (2) hexyle sont évaluées plus lumineuses que les formules ne contenant pas cet ingrédient. En effet, les formules contenant la dispersion d'oxychlorure de bismuth dans hydroxystéarate d'éthyl (2) hexyle réfléchissent plus la lumière, de façon homogène et continue.

Cet effet lumineux est intensifié avec l'augmentation de la quantité de cet ingrédient dans les formules.

### Exemple 2 : Concealer pour masquer les irrégularités visibles du visage

| Nom | 2F (invention) | 2E (invention) | 2 D (comparatif) |
|---|---|---|---|
| Magnésium sulfate | 0,6 | 0,6 | 0,6 |
| Hectorite modifiée Distearyl Dimethyl Ammonium (Bentone 38 VCG de Elementis) | 0,6 | 0,6 | 0,6 |
| Micro-Sphères Chlorure de Vinylidene/Acrylonitrile/PMMA à L'Iso-Butane expansées (Expancel 551 de Expancel) | 0,4 | 0,4 | 0,4 |
| Phenyl trimethicone | 1,2 | 1,2 | 1,2 |
| Conservateurs | 2,18 | 2,18 | 2,18 |
| Pigments : Oxydes de fer jaune, rouge et noir Enrobés de Stearoyl Glutamate d'Aluminium et Oxyde de titane Anatase enrobé de Stearoyl Glutamate d'Aluminium (références NAl-C33-9001-10 NAl-C33-8001-10, NAl-C33-7001-10 et NAl-TAO-77891 de MIYOSHI KASEI de la société MIYOSHI KASEI) | 3 | 7,2 | 11,34 |
| Neopentanoate D'Isodecyle Désodorise | 1,2 | 1,2 | 1,2 |
| Dispersion d'oxychlorure de bismuth dans hydroxystéarate d'éthyl (2) hexyle (70:30) (Timiron Liquid Silver^{®}) de Merck | 15 | 10,8 | 0 |
| Isoparaffine (6-8 Moles D'Isobutylène) Hydrogenée (Parleam de Nof Corporation | 10 | 10 | 10 |
| Cyclopenta dimethylsiloxane (DOW CORNING 245 FLUID de Dow Corning) | 14,5 | 14,5 | 18 |
| Eau | 38,32 | 37,32 | 40,48 |
| Glycérine | 5 | 5 | 5 |
| Propylène glycol | 3 | 3 | 3 |
| Mono-Isostearate de Sorbitane (ARLACEL 987 de Croda) | 5 | 6 | 6 |

### Mode opératoire

On procède comme décrit à l'exemple 1.

### Evaluation maquillage:

On évalue le résultat maquillage des compositions comme décrit à l'exemple 1.

Les formules contenant la dispersion d'oxychlorure de bismuth dans hydroxystéarate d'éthyl (2) hexyle sont évaluées plus lumineuses que les formules ne contenant pas cet ingrédient : elles réfléchissent plus la lumière, de façon homogène et continue.
Cet effet lumineux est intensifié avec l'augmentation de la quantité de cet ingrédient dans les formules.

### Exemple 3 : Emulsion pigmentée

| Nom | 3H (invention) | 3G (comparatif) |
|---|---|---|
| Sulfate de magnésium | 1 | 1 |
| Esters D'Acides Gras Végétaux,lso-Stearique,Adipique de Glyceryle (Softisan 649 de Sasol) | 0,2 | 0,2 |
| Pigments : Oxydes de fer jaune, rouge et noir Enrobés de Stearoyl Glutamate d'Aluminium et Oxyde de titane Anatase enrobé de Stearoyl Glutamate d'Aluminium (références NAl-C33-9001-10 NAl-C33-8001-10, NAl-C33-7001-10 et NAl-TAO-77891 de MIYOSHI KASEI de la société MIYOSHI KASEI) | 11 | 11 |
| Conservateurs | 1,25 | 1,25 |
| 4-Methoxycinnamate de 2-Ethyl Hexyle protégé (Parsol MCX de DSM Nutritional Products) | 5 | 5 |
| Nacres | 0,04 | 0,44 |
| HDI/Trimethylol hexyllactone crosspolymer | 5 | 5 |
| Dispersion d'oxychlorure de bismuth dans hydroxystéarate d'éthyl (2) hexyle (70:30) (Timiron Liquid Silver^{®}) de Merck | 0,5 | 0 |
| Cyclopenta dimethylsiloxane (DOW CORNING 245 FLUID de Dow | 15 | 15 |
| Corning) | | |
| Phenyl trimethicone | 1 | 1 |
| Dimethicone | 5 | 5 |
| Poly Dimethylsiloxane Oxyethylène (DP: 70 - Viscosite: 500 CST) | 2,5 | 2,5 |
| Dimethicone et dimethicone/polyglycerin-3 crosspolymer | 9 | 9 |
| Dimethicone et dimethicone/PEG-10/15 crosspolymer | 7,65 | 7,65 |
| Glycérine | 7 | 7 |
| Eau | 26,86 | 26,96 |
| Propylene glycol | 1 | 1 |
| Pentylène glycol | 1 | 1 |

### Mode opératoire

On procède comme décrit à l'exemple 1

### Evaluation maquillage :

On évalue le résultat maquillage des compositions comme décrit à l'exemple 1.
Les formules contenant la dispersion d'oxychlorure de bismuth dans hydroxystéarate d'éthyl (2) hexyle sont évaluées plus lumineuses que les formules ne contenant pas cet ingrédient : elles réfléchissent plus la lumière, de façon homogène et continue.
Ladite formule contenant la dispersion d'oxychlorure de bismuth dans hydroxystéarate d'éthyl (2) hexyle est plus claire en teinte et plus couvrante. Elle apporte plus d'éclat au visage avec un rendu légèrement plus satiné. Elle est aussi plus unifiante en couleur et estompe plus le relief.

### Exemple 4: Emulsion E/Si

| | | | |
|---|---|---|---|
| | 4A (comparatif) | 4B (invention) | 4C (invention) |
| SOLUTION DE MALTOSE HYDROGENEE | 0,5 | 0,47 | 0,45 |
| TALC: SILICATE DE MAGNESIUM MICRONISE (GRANULOMETRIE: 5 MICRONS) (CI: 77718) | 0,5 | 0,47 | 0,45 |
| PERHYDROSQUALENE VEGETAL | 1 | 0,95 | 0,9 |
| RAFFINEE | | | |
| OXYDE DE FER JAUNE ENROBE DE STEAROYL GLUTAMATE D'ALUMINIUM (3%) NAl-C33-9001-10 de MIYOSHI KASEI | 1,63 | 1,55 | 1,45 |
| OXYDE DE FER ROUGE ENROBE DE STEAROYL GLUTAMATE D'ALUMINIUM (3%) NAl-C33-8001-10 de MIYOSHI KASEI | 0,29 | 0,27 | 0,261 |
| OXYDE DE FER NOIR ENROBE DE STEAROYL GLUTAMATE D'ALUMINIUM (3%) NAl-C33-7001-10 de MIYOSHI KASEI | 0,13 | 0,12 | 0,12 |
| OXYDE DE TITANE ANATASE ENROBE DE STEAROYL GLUTAMATE D'ALUMINIUM (97/3) (CI: 77891) (NAl-TAO-77891 de MIYOSHI KASEI) | 9,95 | 9,45 | 8,95 |
| POLY DIMETHYLSILOXANE A GROUPEMENTS ALPHA-OMEGA OXYETHYLENE / OXYPROPYLENE EN SOLUTION DANS CYCLOPENTASILOXANE ABIL EM 97^{®} de Evonik Goldschmidt | 1 | 0,95 | 0,9 |
| PHENYL TRIMETHYLSILOXY TRISILOXANE (VISCOSITE: 20 CST-PM: 372) DOW CORNING 556 COSMETIC GRADE FLUID de Dow Corning | 2 | 1,9 | 1,8 |
| POLY DIMETHYLSILOXANE OXYETHYLENE (DP: 70 - VISCOSITE: 500 CST) KF 6017^{®} de Shin Etsu | 2 | 1,9 | 1,8 |
| P-METHOXY-4 CINNAMATE D'ETHYL-2 HEXYLE STABILISE (BHT 0,1 %) PARSOL MCX XR de DSM NUTRITIONAL PRODUCTS | 3 | 2,85 | 2,7 |
| Dispersion d'oxychlorure de bismuth dans hydroxystéarate d'éthyl (2) hexyle 70 :30 (Timiron Liquid Silver^{®}) de Merck | 0 | 5 | 10 |
| MICRO-SPHERES DE NYLON-12 (GRANULOMETRIE: 5 MICRONS) | 0,5 | 0,475 | 0,45 |
| ALCOOL ETHYLIQUE 96 DEGRES DENATURE | 13 | 12,35 | 11,7 |
| 1,3-BUTYLENE GLYCOL | 3 | 2,85 | 2,7 |
| SULFATE DE MAGNESIUM | 0,7 | 0,66 | 0,63 |
| EAU DESIONISEE MICROBIOLOGIQUEMENT PROPRE | 36,15 | 34,34 | 32,53 |
| CYCLOPENTA DIMETHYLSILOXANE (DOW CORNING 245 FLUID^{®} de Dow Corning) | Qsp 100 | Qsp 100 | Qsp 100 |

### Mode opératoire

On introduit la dispersion d'oxychlorure de bismuth dans hydroxystéarate d'éthyl (2) hexyle dans la phase grasse. On prépare la phase aqueuse, puis, en utilisant une défloculeuse sur Rayneri, on procède à l'émulsion, sous forte agitation et à température ambiante. Puis, sous agitation modérée, on procède à l'introduction de l'alcool.

### Evaluation maquillage :

Les compositions sont appliquées sur des panels de 10 femmes de 25 à 55 ans ayant une peau mixte à tendance grasse. Les évaluations se font par maquillage du visage desdites femmes (auto-évaluations, évaluations par les formulateurs, évaluation par des esthéticiennes). Les formules sont donc comparées entre elles sur les critères de maquillage et notamment la luminosité.

Les formules contenant la dispersion d'oxychlorure de bismuth dans hydroxystéarate d'éthyl (2) hexyle sont évaluées plus lumineuses que la formule ne contenant pas cet ingrédient. En effet, les formules contenant la dispersion d'oxychlorure de bismuth dans hydroxystéarate d'éthyl (2) hexyle réfléchissent plus la lumière, de façon homogène et continue. Cet effet lumineux est intensifié avec l'augmentation de la quantité de cet ingrédient dans les formules.

De plus, la texture fluide pénètre en laissant une sensation douce et soyeuse sous les doigts, pour un rendu maquillage discret/naturel. Le teint est lumineux et unifié et les petites imperfections de couleurs (rougeurs, cernes) sont atténuées. Le fond de teint capte la lumière pour une impression de peau détendue.

### Exemple 5 : Emulsion eau-dans-huile pigmentée

On a préparé la composition suivante et évalué son effet après application sur la peau de modèles.

| | |
|---|---|
| Talc | 0,5% |
| Dioxyde de titane enrobé de stearoyl glutamate d'aluminium | 7,0% |
| Pigment composite (dioxyde de titane/ FD&C Blue 1 A1 lake)⁽¹⁾ | 0,1% |
| Pigment composite (dioxyde de titane/ Red n°28 lake/ Red 7)⁽²⁾ | 0,05% |
| Oxydes de fer jaune, rouge et noir respectivement enrobés de stearoyl glutamate d'aluminium | 4,0% |
| Pré-dispersion d'oxychlorure de bismuth dans hydroxystéarate d'éthyl (2) hexyle (70 :30) (Biron® Liquid Silver de Merck) | 3,0% |
| Cyclopentadimethylsiloxane (Dow Corning 245 Fluid) | 22,5% |
| Phényl Trimethicone | 5,0% |
| BIS-PEG/PPG-14/14 DIMETHICONE (and) CYCLOPENTASILOXANE⁽³⁾ | 1,2% |
| PEG-10 DIMETHICONE ⁽⁴⁾ | 2,3% |
| DIMETHICONE (and) DIMETHICONE/POLYGLYCERIN-3 CROSSPOLYMER⁽⁵⁾ | 3,0% |
| Alcool éthylique dénaturé | 9,0% |
| Eau désionisée microbiologiquement propre | qsp 100% |

(1) Pigment composite constitué d'un noyau inorganique de dioxyde de titane de taille moyenne 20 nm et de surface spécifique pour 50 m²/g, de laque organique de dénomination FD&C Blue 1 Al lake et réalisé avec un liant polyméthylhydrogénosiloxane (proportions 58.1/40.7/1.2)
(2) Pigment composite constitué d'un noyau inorganique de dioxyde de titane de taille moyenne 20 nm et de surface spécifique pour 50 m²/g, de laque organique de dénomination Red n°28 lake et de pigment organique Red 7 et réalisé avec un liant polyméthylhydrogénosiloxane
(3) Abil EM 97 de Evonik Goldschmidt
(4) KF 6017 de Shin Etsu
(5) (3) KSG710 de Shin Etsu

### Mode opératoire

Introduire toutes les matières premières à l'exception de l'alcool éthylique à température ambiante dans un bécher inox.

Mélanger grossièrement à la spatule.Turbiner à grande vitesse (3000 tr/mn) à l'aide d'un Moritz (petite turbine) jusqu'à l'obtention d'une émulsion (8mn). Ajouter ensuite l'alcool éthylique et garder la même agitation 2 à 3mn supplémentaires.

Après application sur la peau, et comparativement à une peau nue, la composition de l'invention améliore la lumière, l'homogénéité et la finesse du grain de la peau avec un effet bonne mine avantageux. L'oxychlorure de bismuth dispersé dans l'hydroxystéarate d'éthyl (2) hexyle apporte un effet correcteur et unifiant du teint ainsi qu'un effet réflecteur de lumière ; les pigments composites apportent un effet désaturant , un effet correcteur de couleur anti-teint terne.

Le teint retrouve ainsi sa luminosité naturelle, avec un résultat maquillage qui ne marque pas les traits. La composition permet à la peau de réfléchir davantage la lumière, de façon homogène et continue. Le teint paraît plus jeune, avec plus d'éclat après application de la composition selon l'invention, comparativement à la peau nue .

## Revendications

1. Composition cosmétique sous la forme d'une émulsion pour application topique sur les matières kératiniques, en particulier la peau, comprenant, dans un milieu physiologiquement acceptable :
(i) au moins une dispersion d'oxychlorure de bismuth dans de l'hydroxystéarate d'éthyl (2) hexyle, et
(ii) au moins un agent à effet de flou (soft focus) et
(iii) au moins une huile siliconée phénylée.

2. Composition selon la revendication 1, **caractérisée en ce que** la teneur totale en oxychlorure de bismuth et huile associée va de 0.01 à 15%, préférentiellement de 0.5% à 10% en poids par rapport au poids total de ladite composition, notamment avec un rapport pondéral oxychlorure de bismuth/ huile associée allant de 2 à 3.

3. Composition selon l'une quelconque des revendications précédentes, **caractérisée en ce que** l'agent à effet de flou est choisi parmi les charges minérales, les charges organiques, les agents de colorations composites, les élastomères de silicone, et leurs mélanges.

4. Composition selon la revendication précédente, **caractérisée en ce que** l'agent à effet de flou est choisi parmi les poudres de polytétrafluoroéthylène, les poudres de polyuréthane, les microcires de Carnauba, les microcires de cire synthétique, les poudres de résine de silicone , les particules hémisphériques creuses de silicone , les poudres de copolymères acryliques, les microsphères de vinylidène/acrylanitrile/méthacrylate de méthylène expansées , les poudres de polyéthylène, notamment comprenant au moins un copolymère éthylène/acide acrylique, les poudres d'organopolysiloxane élastomérique réticulé, les poudres d'organopolysiloxane élastomérique réticulé enrobées de résine de silicone, les poudres de polyamide, les poudres de silice et silicates, notamment d'alumine, le talc de taille moyenne en nombre inférieure ou égale à 3 microns, les composites silice/TiO₂ , les particules de sulfate de baryum, les particules de nitrure de bore, les particules de silice traitées en surface par une cire minérale 1 à 2 % , les microsphères de silice amorphe, les micro-billes de silice , les poudres composites de talc/TiO₂/alumine/silice, les pigments composites de séricite/TiO₂/oxyde de fer brun/silice, les élastomères de silicone, et leurs mélanges.

5. Composition selon la revendication précédente, **caractérisée en ce que** l'agent à effet de flou est choisi parmi un pigment composite de séricite/TiO₂/oxyde de fer brun/silice, une poudre de polyuréthane, une élastomère de silicone, et leurs mélanges

6. Composition selon l'une quelconque des revendications 1 à 5, **caractérisée en ce qu'**elle est sous la forme d'une émulsion eau-dans-huile (E/H).

7. Composition selon l'une quelconque des revendications 1 à 6, comprenant une phase aqueuse en une teneur variant de 10 à 80 %, et plus particulièrement de 20 à 60 % en poids, notamment de 30 à 50% en poids par rapport au poids total de la composition.

8. Composition selon l'une quelconque des revendications 1 à 7 comprenant une phase aqueuse formant de 30 à 70% en poids par rapport au poids total de ladite composition, et au moins 5% en poids, par rapport au poids total de la composition d'au moins un mono-alcool en C₂ à C₈

9. Composition selon l'une quelconque des revendications précédentes, **caractérisée en ce qu'**elle contient en outre au moins une matière colorante pulvérulente.

10. Composition selon l'une quelconque des revendications précédentes, **caractérisée en ce qu'**elle contient en outre des charges additionnelles.

11. Composition selon l'une quelconque des revendications précédentes, **caractérisée en ce qu'**il s'agit d'un produit de soin et/ou de maquillage de la peau, en particulier de la peau du corps ou du visage.

12. Composition selon la revendication précédente, **caractérisée en ce qu'**il s'agit d'un fond de teint ou d'un correcteur de teint.

13. Procédé cosmétique de soin et/ou de maquillage des matières kératiniques comprenant l'application, sur lesdites matières kératiniques, en particulier sur la peau, d'une composition telle que définie selon l'une quelconque des revendications 1 à 12.

14. Procédé cosmétique selon la revendication précédente, **caractérisé en ce que** l'application de ladite composition sur la peau, notamment du visage, apporte à celle-ci un effet lumineux et de la couvrance .

## Patentansprüche

1. Kosmetische Zuaammensetzung in Form einer émulsion zur topischen Abwendung auf Keratinmaterialien, insbesondere die Haut, umfassend in einem physiologisch unbedenkliche Medium:
(i) mindestens eine Dispersion von Bismutoxidchlorid in 2-Ethylhexylhydroxystearat und
(ii) mindestens ein Mittel mit Weichzeichner-Effekt (Soft-Focus-Effekt) und
(iii) mindestens ein Phenylsilikonöl.

2. Zusammensetzung nach dem Anspruch 1, **dadurch gekennzeichnet, dass** der Gesamtgehalt von Bismutoxidchlorid und assoziiertem Öl im Bereich von 0,01 bis 15 Ges.-%, vorzugsweise von 0,5 bis 10 Ges.-%, bezogen auf das Gesamtgewicht der Zusammensetzung, insbesondere mit einem Gewichtesverhältnis von Bismutoxidchlorid zu assoziiertem Öl im Bereich von 2 bis 3, liegt.

3. Zusammensetzung nach einem der vorhergehenden Ansprüche, **dadurch gekennzeichnet, dass** das Mittel mit Weichzeichner-Effekt aus anorganischen Füllstoffen, organischen Füllstoffen, Verbundfarbmitteln, Silikonelastomeren und Mischungen davon ausgewählt ist.

4. Zusammensetzung nach dem vorhergehenden Anspruch, **dadurch gekennzeichnet, dass** das Mittel mit Weichzeichner-Effekt aus Polytetrafluorethylen-Pulvern, Polyurethan-Pulvern, Carnauba-Mikrowachsen, Mikrowachsen aus synthetischen Wachs, Silikonharze-Pulvern, hohlen halbkugelförmigen Bilikonteilchen, Acrylcopolymer-Pulvern, expandierten Vinyliden/Acrylonitril/Methylenmethacrylat-Mikrokugeln, Polyethylen-Pulvern, die insbesondere mindestens ein Ethylen/AcrylsäureCopolymer umfassen, Pulvern von vernetztem elastomerem Organopolysiloxan, Pulvern aus vernetztem elastomerem Organopolysiloxan, die mit Silikonharz überzogen sind, Polyamid-Pulvern, Siliciumdioxid- und Silikat-Pulvern, insbesondere Aluminiumoxid-Pulver, Talk mit einer zahlenmittleren Größe kleiner gleich 3 Mikron, Siliciumdioxid/TiO₂-Verbunden, Bariumsulfatteilchen, Bornitridteilchen, mit einem anorganischen Wachs in einer Menge von 1 bis 2% obertlächenbehandelten Siliciumdioxidteilchen, Mikrokugeln aus amorphem Siliziumdioxid, Siliciumdioxid-Mikroperlen, Talk/TiO₂/Aluminiumoxid/Siliciumdioxid-Verbundpulvern, Sericit/TiO₂/Braüneisenbxid/Silzciumdioxid-Verbundpigmenten, Silikonelastomeren und Mischungen davon ausgewählt ist.

5. Zusämmensetzung nach dem vorhergehenden Anspruch, **dadurch gekennzeichnet, dass** das Mittel mit Weichzeichner-Effekt aus einem Sericit/TiO₂/Brauneisenoxid/Siliciumdioxid-Verbundpigment, einem Polyurethan-Pulver, einem Silikonelastomer und Mischungen davon ausgewählt ist.

6. Zusammensetzung nach einem der Ansprüche 1 bis 5, **dadurch gekennzeichnet, dass** sie in Form einer Wasser-in-Öl-Emulsion (W/O-Emulsion) vorliegt.

7. Zusammensetzung nach einem der Ansprüche 1 bis 6, umfassen eine wässerige Phase in einem Gehalt im Bereich von 10 bis 80 Gew.-% und spezieller 20 bis 60 Gew.-%, insbesondere 30 bis 50 Gew.-%, bezogen auf das Gesamtgewicht der Zusammensetzung, umfasst.

8. Zuaammensetzung nach einem der Ansprüche 1 bis 7, umfassen eine wässrige Phase, die 30 bis 70 Gew.-%, bezogen auf das Gesamtgewicht der Zusammensetzung, ausmacht, und mindestens 5 Gew.-%, bezogen auf das Gesamtgewicht der Zusammensetzung, mindestens eines C₂- bis C₈-monoalkohols umfasst.

9. Zusammensetzung nach einem der vorhergehenden Ansprüche, dadurch gekenntzeichnet, dass sie außerdem mindestens ein pulverförmiges Farbmittel enthält.

10. zusammensetzung nach einem der vorhergehenden Ansprüche, **dadurch gekennzeichnet, dass** sie außerdem zusätzliche Füllstoffe enthält.

11. Zusammensetzung nach einem der vorhergehenden Ansprüche, **dadurch gekennzeichnet, dass** es sich um ein Produkt zum Pflegen und/oder Schminken der Haut, insbesondere der Haut des Körpers oder des Gesichts, handelt.

12. Zusammensetzung nach dem vorhergehenden Anspruch, **dadurch gekennzeichnet, dass** es sich um eine Makeup-Grundierung oder ein Produkt für die Teintkorrektur handelt.

13. Kosmetisches Verfahren zum Pflegen und/oder Schminken von Keratinmatexialien, bei dem man auf die Keratinmaterialien, insbesondere auf die Haut, eine Zusammensetzung gemäß einem der Ansprüche 1 bis 12 aufbringt.

14. Kosmetisches Verfahren nach dem vorhergehenden Anspruch, **dadurch gekennzeichnet, dass** das Aufbringen der Zusammensetzung auf die Haut, insbesondere das Gesicht, dieser einen leuchtenden Effekt und Deckung verleiht.

## Claims

1. Cosmetic composition in the form of an emulsion for topical application to keratin materials, in particular the skin, comprising, in a physiologically acceptable medium:
(i) at least one dispersion of bismuth oxychloride in 2-ethylhexyl hydroxystearate, and
(ii) at least one soft-focus agent, and
(iii) at least one phenyl silicone oil.

2. Composition according to Claim 1, **characterized in that** the total content of bismuth oxychloride and associated oil ranges from 0.01% to 15%, preferentially from 0.5% to 10% by weight relative to the total weight of said composition, in particular with a bismuth oxychloride/associated oil weight ratio ranging from 2 to 3.

3. Composition according to either one of the preceding Claims, **characterized in that** the soft-focus agent is chosen from inorganic fillers, organic fillers, composite colouring agents, silicone elastomers, and mixtures thereof.

4. Composition according to the preceding claim, **characterized in that** the soft-focus agent is chosen from polytetrafluoroethylene powders, polyurethane powders, carnauba microwaxes, synthetic wax microwaxes, silicone resin powders, hollow hemispherical silicone particles, acrylic copolymer powders, expanded vinylidene/acrylonitrile/methylene methacrylate microspheres, polyethylene powders, especially comprising at least one ethylene/acrylic acid copolymer, crosslinked elastomeric organopolysiloxane powders, crosslinked elastomeric organopolysiloxane powders coated with silicone resin, polyamide powders, powders of silica and silicates, especially of alumina, talc having a number-average size of less than or equal to 3 microns, silica/TiO2 composites, barium sulphate particles, boron nitride particles, silica particles surface-treated with an inorganic wax at 1 to 2%, amorphous silica microspheres, silica microbeads, talc/TiO₂/alumina/silica composite powders, sericite/TiO₂/brown iron oxide/silica composite pigments, silicone elastomers, and mixtures thereof.

5. Composition according to the preceding Claim, **characterized in that** the soft-focus agent is chosen from a sericite/TiO₂/brown iron oxide/silica composite pigment, a polyurethane powder, a silicone elastomer, and mixtures thereof.

6. Composition according to any one of Claims 1 to 5, **characterized in that** it is in the form of a water-in-oil (W/O) emulsion.

7. Composition according to any one of Claims 1 to 6, comprising an aqueous phase in a content ranging from 10% to 80%, and more particularly from 20% to 60% by weight, especially from 30% to 50% by weight relative to the total weight of the composition.

8. Composition according to any one of Claims 1 to 7, comprising an aqueous phase forming from 30% to 70% by weight relative to the total weight of said composition, and at least 5% by weight, relative to the total weight of the composition, of at least one C₂ to C₈ monoalcohol.

9. Composition according to any one of the preceding claims, **characterized in that** it also contains at least one pulverulent colorant.

10. Composition according to any one of the preceding claims, **characterized in that** it also contains additional fillers.

11. Composition according to any one of the preceding claims, **characterized in that** it is a product for caring for and/or making up the skin, in particular the skin of the body or of the face.

12. Composition according to the preceding claim, **characterized in that** it is a foundation or a complexion corrector.

13. Cosmetic method for caring for and/or making up keratin materials, comprising the application to said keratin materials, in particular to the skin, of a composition as defined in any one of Claims 1 to 12.

14. Cosmetic method according to the preceding claim, **characterized in that** the application of said composition to the skin, especially facial skin, provides the latter with a luminous effect and coverage.
